# EUROPEAN PATENT SPECIFICATION

(11) **EP 3 956 026 B1**
(45) Date of publication and mention of the grant of the patent: **07.06.2023**
(21) Application number: 20722496.5
(22) Date of filing: 17.04.2020
(51) Int. Cl.: A61P 25/14, A61K 48/00, A61K 31/00, A61K 31/713

(54) **P16INK4A INHIBITOR FOR PREVENTING OR TREATING HUNTINGTON'S DISEASE**
P16INK4A-INHIBITOR ZUR VORBEUGUNG ODER BEHANDLUNG DER HUNTINGTON-KRANKHEIT
INHIBITEUR P16INK4A POUR LA PRÉVENTION OU LE TRAITEMENT DE LA MALADIE DE HUNTINGTON

(30) Priority: 19.04.2019 EP 19305516
(43) Date of publication of application: 23.02.2022
(73) Proprietor: SORBONNE UNIVERSITE, 75006 Paris (FR); Centre national de la recherche scientifique, 75016 Paris (FR); Assistance Publique Hôpitaux de Paris, 75004 Paris (FR); Buck Institute for Research on Aging, Novato, CA 94945 (US)
(72) Inventor: NERI, Christian, 75013 Paris (FR); FARINA, Francesca, Paris 75014 (FR); VOISIN, Jessica, 94600 Choisy-le-Roi (FR); ELLERBY, Lisa, San Aselmo, California 94960 (US); DANCOURT, Julia, 75013 Paris (FR)
(74) Representative: Icosa
(86) International application number: PCT/EP2020/060904
(87) International publication number: WO 2020/212597

(56) References cited:
- WO-A2-2004/047872
- WO-A2-2006/053201
- US-A1- 2010 158 869
- SEBASTIAN AGUIAR ET AL: "RNAi mechanisms in Huntington's disease therapy: siRNA versus shRNA", TRANSLATIONAL NEURODEGENERATION, BIOMED CENTRAL LTD, LONDON, UK, vol. 6, no. 1, 27 November 2017 (2017-11-27), pages 1-10, XP021250900, DOI: 10.1186/S40035-017-0101-9
- PFISTER E L ET AL: "Huntington's disease: Silencing a brutal killer", EXPERIMENTAL NEUROLOGY, ELSEVIER, AMSTERDAM, NL, vol. 220, no. 2, 1 December 2009 (2009-12-01), pages 226-229, XP026762272, ISSN: 0014-4886, DOI: 10.1016/J.EXPNEUROL.2009.09.017 [retrieved on 2009-09-25]
- DARREN J. BAKER ET AL: "Clearance of p16Ink4a-positive senescent cells delays ageing-associated disorders", NATURE, vol. 479, no. 7372, 1 November 2011 (2011-11-01), pages 232-236, XP055074325, ISSN: 0028-0836, DOI: 10.1038/nature10600

## Description

### FIELD OF INVENTION

The present invention relates to the treatment of Huntington's Disease.

### BACKGROUND OF INVENTION

According to the U. S National Library of Medicine, Huntington's disease is a progressive brain disorder that causes uncontrolled movements, emotional problems, and loss of thinking ability (cognition).

Adult-onset Huntington's disease, the most common form of this disorder, usually appears in a person's thirties or forties. Early signs and symptoms can include irritability, depression, small involuntary movements, poor coordination, and trouble learning new information or making decisions. Many people with Huntington's disease develop involuntary jerking or twitching movements known as chorea. As the disease progresses, these movements become more pronounced. Affected individuals may have trouble walking, speaking, and swallowing. People with this disorder also experience changes in personality and a decline in thinking and reasoning abilities. Individuals with the adult-onset form of Huntington disease usually live about 15 to 20 years after signs and symptoms begin.

A less common form of Huntington's disease known as the juvenile form begins in childhood or adolescence. It also involves movement problems and mental and emotional changes. Additional signs of the juvenile form include slow movements, clumsiness, frequent falling, rigidity, slurred speech, and drooling. School performance declines as thinking and reasoning abilities become impaired. Seizures occur in 30 percent to 50 percent of children with this condition. Juvenile Huntington disease tends to progress more quickly than the adult-onset form; affected individuals usually live 10 to 15 years after signs and symptoms appear.

Despite the fact that HD is a neurodegenerative disease (ND) such as Alzheimer's disease (AD), Parkinson's disease (PD), Amyotrophic lateral sclerosis (ALS), Prion disease and Dentatorubral-pallidoluysian atrophy (DRPLA), Frontotemporal dementias (FTDs), Spinocerebellar Ataxias (SCAs) and that many of the NDs, share common features and molecular mechanisms, no link has ever been shown between HD and cellular senescence.

Cellular senescence is a process that imposes permanent proliferative arrest on cells in response to various stressors. It has historically been viewed as having a role in complex biological processes such as ageing and age-related disorders. Cellular senescence is associated with cells attempting to repair their cellular components (cell repair), notably the attempt of cells to repair DNA damage, which may involve cell cycle arrest or re-entry into the cell cycle. Cellular senescence may result from prolonged though unsuccessful or sub-optimal cell repair. The link between cell repair and cellular senescence applies to dividing cells, including but not only dividing cells of the brain such as astrocytes, oligodendrocytes and microglia, as well as post-mitotic cells, including but not only post-mitotic cells of the brain such as neurons. In post-mitotic cells, cellular senescence is often referred to as a 'cellular senescence like status' or' senescence response'. In both case, cellular senescence features may include cellular vulnerability to external stressors as well as secretion of molecules that are harmful to surrounding cells, such as inflammatory cytokines. A major regulator of cell cycle arrest is p16^{INK4a,} also a major inducer and important marker of cellular senescence.

The present invention results from the serendipitous discovery of the role of p16^{INK4a} in the prevention or treatment of HD.

### SUMMARY

The present invention deals with a p16^{INK4a} inhibitor for use in preventing and/or treating Huntington's disease (HD). The invention is defined by the appended claims.

In a particular aspect of the invention said inhibitor is a nucleic acid, a peptide, a small compound molecule.

In a further aspect of the invention, the p16^{INK4a} inhibitor is a nucleic acid that encodes an RNA interfering with p16^{INK4a} such as a siRNA, shRNA, micro RNA, non-coding RNA, deoxyribosyme, antisense oligonucleotide, ribozymes DNAzymes, modified or synthetic DNA or RNA degradation-resistant polynucleosides amides, peptide nucleic acids (PNAs), locked nucleic acids (LNAs), other nucleobase-containing polymers, aptamers or a polynucleotide targeted gene editing or anycombination thereof.

In a further aspect of the invention, the p16^{INK4a} inhibitor is a peptide chosen among the group comprising a ligand, an inhibitor of kinase, a small compound molecule such as PPARγ antagonist or such as a retinoid X receptor (RXR) antagonist small molecule SIRT1 activators, compound able to stimulate the activity of FOXO factors, AMPK activators

In a further aspect of the invention, the p16^{INK4a} inhibitor is a ligand, said ligand being an antibody, Fab, Fab', F(ab')2, Fv, dsFv, scFv, diabody, triabody, tetrabody, an aptamer or VHH domain.

According to another aspect, the invention deals with a composition for use in treating or preventing HD wherein said composition comprises at least one p16^{INK4a} inhibitor as above described.

In a further aspect of the invention, the composition is a pharmaceutical composition and further comprises at least one pharmaceutically acceptable excipient.

In a further aspect of the invention, the composition contains a nucleic acid sequence encoding a peptide for cell-specific targeting and/or contains a nucleic acid enabling a cell-specific expression.

In a further aspect of the invention, the composition further comprises one or more active agent(s) for treating HD and/or side effects of said active agent(s).

In a still further aspect, the invention deals with a medicament comprising at least one p16^{INK4a} inhibitor for use as above described.

In a specific aspect, the p16^{INK4a} inhibitor or the composition for use according to anyone of the preceding claims administered to the subject in a therapeutically effective amount.

In a further aspect of the invention the p16^{INK4a} inhibitor the composition or the medicament is administered to the subject in a therapeutically effective amount.

In a further aspect of the invention the subject is diagnosed with HD, presents a genetic predisposition to HD or is affected,

In a more preferred aspect of the invention, the subject is diagnosed with HD.

### DEFINITIONS

"**p16^{INK4}**", as used herein, is the principal member of the Ink4 family of CDK (Cyclin-Dependent Kinase) inhibitors. It is encoded by the CDKN2A gene localized on chromosome 9p21 within the INK4a/ARF locus, which encodes for two different proteins with different promoters: p16^{Ink4a} and p19^{ARF}. It contributes to the regulation of cell cycle progression by inhibiting the S phase. In addition to the action of p16^{Ink4a} in cell cycle regulation, this protein has also been implicated in other processes, such as apoptosis, cell invasion and angiogenesis.

"**p16^{INK4}inhibitor**" as used herein, refers to any molecule, compound or substance that, when administered to a subject, leads to a partial or complete reduction of the normal physiological activity of p16^{INK4}. By normal physiological activity of p16^{INK4} is meant the physiological activity of these CDK inhibitors.

"**Huntington's Disease**", as defined by Mayo Clinic and as used herein, refers to "an inherited disease that is associated with CAG repeat expansion in the gene huntingtin, or other genetic causes that produces HD clinical phenocopies, and that may cause the progressive break-down (cell dysfunction possibly followed by cell degeneration) of nerve cells, neurons and other cells in the brain. Huntington's disease has a broad impact on a person's functional abilities and usually results in movement, thinking (cognitive) and psychiatric disorders.

"**Expression**", as used herein, refers to the expression of a gene. Expression of a gene may be determined at the protein level by ways of, e.g., immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA) and the like. Alternatively, expression of a gene may be determined at the mRNA level, by ways of, *e.g*., RT-PCR, RT-qPCR (wherein qPCR stands for quantitative PCR), hybridization techniques such as, for example, Northern Blot, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

"**Polynucleotide**", "**nucleotide sequence**", "**nucleic acid**", "**nucleic acid molecule**", "**nucleic acid sequence**" and "**oligonucleotide**" refer to a series of nucleotide bases (also called "nucleotides") in DNA and RNA, and mean any chain of two or more nucleotides. The polynucleotides can be chimeric mixtures or derivatives or modified versions thereof, single-stranded or double-stranded.

"**Overexpression**" as used herein, refers to the expression of a gene being higher in a sample when compared to a "reference expression level". The reference expression level can be the typical level of expression observed in a similar population of cancer cell among a large group of subjects (typically more than 10, preferably at least 50 or more subjects). The reference expression level can also refer to the level of expression in healthy cells of the same tissue of origin in the same subject or derived from a large group of subjects. The reference expression level can also refer also the level of expression in the cancer cells of a subject at different time points. The person of the art is familiar with the techniques allowing the comparison of gene expression level.

"**Receptor antagonist**", as used herein, refers to any molecule, compound or substance that binds to a receptor and thereby prevents the normal physiological activity which is observed upon binding of its activating ligand (*i.e*., the receptor agonist). A receptor antagonist can, for instance, compete with the binding of the agonist to the receptor.

"**Preventing**" means starting a treatment before the onset of severe symptoms, that is in the pre-symptomatic or pro-dromal phases of Huntington's disease. Preventing means also delay the age at onset of targeted pathologic condition.

"**Treating**" means starting a treatment at any time of the symptomatic HD process in order to stop or slow-down the progression of the disease. As such, "treat" means slow-down (lessen) the targeted pathologic condition or disorder.

"**Therapeutically effective amount**", as used herein, refers to the level or amount of the inhibitor or composition according to the present invention, that is aimed at (but without causing significant negative or adverse side effects to the subject): (1) delaying or preventing the onset of the targeted condition or disorder; (2) slowing down or stopping the progression, aggravation, or deterioration of one or more symptoms of the targeted condition or disorder; (3) bringing about ameliorations of the symptoms of the targeted condition or disorder; (4) reducing the severity or incidence of the targeted condition or disorder; and/or (5) curing the targeted condition or disorder. A therapeutically effective amount of the inhibitor or composition according to the present invention may be administered prior to the onset of the targeted condition or disorder, for a prophylactic or preventive action. Alternatively or additionally, the therapeutically effective amount of the inhibitor or composition according to the present invention may be administered after initiation of the targeted condition or disorder, for a therapeutic action.

"**Subject**", as used herein, refers to a warm-blooded animal, preferably a human. In some embodiments, the subject is a male or female subject. In some embodiments, the subject is an adult (for example, a subject above the age of 18 (in human years) or a child (for example, a subject under the age of 18, more particularly under the age of 15 and more particularly under the age of 10 (in human years). In some embodiments, the subject may be a "**patient**", *i.e*., a subject who/which is awaiting the receipt of or is receiving medical care or was/is/will be the object of a medical procedure according to the methods of the present invention or is monitored for the development of a disease.

### DETAILED DESCRIPTION

Huntington's disease subject develops signs and symptoms in their 30's or 40's. But the disease may emerge earlier or later in life. When the disease develops before age 20, the condition is called "Juvenile Huntington's Disease". An earlier emergence of the disease often results in a somewhat different set of symptoms and faster disease progression. Medications are available to help manage the symptoms of Huntington's disease, but treatments can't prevent the physical, mental and behavioral decline associated with cellular dysfunction and degeneration in the brain.

The present invention responds to this need by making use of a p16^{INK4a} inhibitor or of a composition comprising said p 16^{INK4a} inhibitor as active agent to prevent and/or treat HD.

The present invention makes use of a p16^{INK4a} inhibitor or of a composition comprising said p16^{INK4a} inhibitor as active agent to prevent and/or treat HD.

According to a first embodiment, this invention relates to a p16^{INK4a} inhibitor, for use in preventing and/or treating Huntington disease.

p16^{Ink4a} is a protein involved in regulation of the cell cycle. p16^{Ink4a} is the principal member of the Ink4 family of CDK inhibitors. It is codified by a gene localized on chromosome 9p21 within the INK4a/ARF locus. p16 is an inhibitor of cyclin-dependent kinases (CDK). Expression of p16^{Ink4a} markedly increases with ageing in most mouse tissues and in human skin and kidney tissues, suggesting the importance of this tumor suppressor in ageing and senescence. In addition, p16^{Ink4a} overexpression has been reported in senescent fibroblasts, in response to oxidative stress, DNA damage and changes in chromatin structure. Nonetheless, a complete understanding of the signals that trigger senescence is currently lacking, and although p16^{Ink4a} appears to be one of the principal factors in senescence, more information is needed to ascertain the exact role of each factor in this process.

In a particular aspect, the p16^{INK4a} inhibitor for use according to the present invention is a nucleic acid, a polypeptide or a small compound molecule.

In a more specific aspect of the invention, the p16^{INK4a} inhibitor for use according to the present invention is a nucleic acid sequence that encodes an RNA interfering with p16^{INK4a} such as a siRNA, shRNA, micro RNA, non-coding RNA, deoxyribosyme, antisense oligonucleotide, ribozymes DNAzymes, modified or synthetic DNA or RNA degradation-resistant polynucleosides amides, peptide nucleic acids (PNAs), locked nucleic acids (LNAs), other nucleobase-containing polymers, or aptamers.

The nucleic acid molecule or sequence of the invention refer to a series of nucleotide bases (also called "nucleotides") in DNA and RNA, and mean any chain of two or more nucleotides. The polynucleotides can be chimeric mixtures or derivatives or modified versions thereof, single-stranded or double-stranded.

The oligonucleotide can be modified at the base moiety, sugar moiety, or phosphate backbone, e.g., to improve stability of the molecule, its hybridization parameters, *etc.* The antisense oligonuculeotide may comprise a modified base moiety which is selected from the group including, but not limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, β-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, β-D-mannosylqueosine, 5'-methoxycarboxymethyluracil, 5-methoxyuracil, 2-methylthio-N6-isopentenyladenine, wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid, 5-methyl-2-thiouracil, 3-(3-amino-3-*N*-2-carboxypropyl) uracil, a thio-guanine and 2,6-diaminopurine.

A nucleotide sequence typically carries genetic information, including the information used by cellular machinery to make proteins and enzymes. These terms include double- or single-stranded genomic and complementary DNA, RNA, any synthetic and genetically manipulated polynucleotide, and both sense and antisense polynucleotides. This includes single- and double-stranded molecules, *i.e*., DNA-DNA, DNA-RNA and RNA-RNA hybrids, as well as "protein nucleic acids" (PNAs) formed by conjugating bases to an amino acid backbone. This also includes nucleic acids containing carbohydrate or lipids.

Exemplary DNAs include, but are not limited to, single-stranded DNA (ssDNA), double- stranded DNA (dsDNA), plasmid DNA (pDNA), genomic DNA (gDNA), complementary DNA (cDNA), antisense DNA, chloroplast DNA (ctDNA or cpDNA), microsatellite DNA, mitochondrial DNA (mtDNA or mDNA), kinetoplast DNA (kDNA), provirus, lysogen, repetitive DNA, satellite DNA, and viral DNA.

Exemplary RNAs include, but are not limited to, single-stranded RNA (ssRNA), double-stranded RNA (dsRNA), small interfering RNA (siRNA), messenger RNA (mRNA), precursor messenger RNA (pre-mRNA), small hairpin RNA or short hairpin RNA (shRNA), microRNA (miRNA), guide RNA (gRNA), transfer RNA (tRNA), antisense RNA (asRNA), heterogeneous nuclear RNA (hnRNA), coding RNA, non-coding RNA (ncRNA), long non-coding RNA (long ncRNA or lncRNA), satellite RNA, viral satellite RNA, signal recognition particle RNA, small cytoplasmic RNA, small nuclear RNA (snRNA), ribosomal RNA (rRNA), Piwi-interacting RNA (piRNA), polyinosinic acid, ribozyme, flexizyme, small nucleolar RNA (snoRNA), spliced leader RNA, viral RNA, and viral satellite RNA.

Polynucleotides described herein may be synthesized by standard methods known in the art, *e.g*., by use of an automated DNA synthesizer (such as those that are commercially available from Biosearch, Applied Biosystems, *etc*.). A number of methods have been developed for delivering antisense DNA or RNA to cells, *e.g*., antisense molecules can be injected directly into the tissue site, or modified antisense molecules, designed to target the desired cells (antisense linked to peptides or antibodies that specifically bind receptors or antigens expressed on the target cell surface) can be administered systemically. Alternatively, RNA molecules may be generated by *in vitro* and *in vivo* transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines. However, it is often difficult to achieve intracellular concentrations of the antisense sufficient to suppress translation of endogenous mRNAs. Therefore, a preferred approach utilizes a recombinant DNA construct in which the antisense oligonucleotide is placed under the control of a strong promoter. The use of such a construct to transfect target cells in the patient will result in the transcription of sufficient amounts of single stranded RNAs that will form complementary base pairs with the endogenous target gene transcripts and thereby prevent translation of the target gene mRNA. For example, a vector can be introduced in vivo such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human, cells. Such promoters can be inducible or constitutive. Any type of plasmid, cosmid, yeast artificial chromosome, or viral vector can be used to prepare the recombinant DNA construct that can be introduced directly into the tissue site.

The polynucleotides may be flanked by natural regulatory (expression control) sequences or may be associated with heterologous sequences, including promoters, internal ribosome entry sites (IRES) and other ribosome binding site sequences, enhancers, response elements, suppressors, signal sequences, polyadenylation sequences, introns, 5'- and 3'-non-coding regions and the like.

In a specific aspect the p16^{Ink4a} inhibitor according to the present invention is the specific construct *INK-ATTAC*, for inducible elimination of p16^{Ink4a}-positive senescent cells upon administration of a drug. The *INK-ATTAC* transgenic construct was made as follows. The FKBP-Casp8 fragment was subcloned from the aP2-ATTAC transgenic construct (Pavani UB, et al. Nature Med. 2005;11:797-803.) and inserted into pBlueScriptII (Stratagene). A 2,617-bp segment of the murine *p16*^{Ink4a} promoter was PCR amplified from BAC DNA to replace the aP2 promoter. An IRES-EGFP fragment was inserted 3' of the ATTAC. This contruct is described in Baker D. J. et al., Nature. 2011 Nov 2; 479(7372): 232-236.

The nucleic acids may also be modified by many means known in the art. Non-limiting examples of such modifications include methylation, "caps", substitution of one or more of the naturally occurring nucleotides with an analog, and internucleotide modifications, such as, for example, those with uncharged linkages (*e.g*., methyl phosphonates, phosphotriesters, phosphoroamidates, carbamates, *etc*.) and with charged linkages (*e.g*., phosphorothioates, phosphorodithioates, *etc*.). Polynucleotides may contain one or more additional covalently linked moieties, such as, for example, proteins (*e.g*., nucleases, toxins, antibodies, signal peptides, poly-1-lysine, *etc*.), intercalators (*e.g*., acridine, psoralen, *etc*.), chelators (*e.g.,* metals, radioactive metals, iron, oxidative metals, etc.), and alkylators. The polynucleotides may be derivatized by formation of a methyl or ethyl phosphotriester or an alkyl phosphoramidate linkage. Furthermore, the polynucleotides herein may also be modified with a label capable of providing a detectable signal, either directly or indirectly. Exemplary labels include radioisotopes, fluorescent molecules, isotopes (*e.g*., radioactive isotopes), biotin and the like.

"**Aptamer**", as used herein, refers to a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence. Possible modifications, uses and advantages of this class of molecules have been reviewed by Jayasena (1999. Clin Chem. 45(9):1628-50). Peptide aptamers consists of a conformationally constrained antibody variable region displayed by a platform protein, such as *E. coli* thioredoxin A, that are selected from combinatorial libraries by two hybrid methods

"**Antisense oligonucleotides**", including antisense RNA molecules and antisense DNA molecules, would act to directly block the translation of 5-HTR1_{D} mRNA by binding thereto and thus preventing protein translation or increasing mRNA degradation, thus decreasing the level of 5-HTR1_{D}, and subsequently, 5-HTR1_{D} activity, in a cell. For example, antisense oligonucleotides of at least about 15 bases and complementary to unique regions of the mRNA transcript sequence encoding 5-HTR1_{D} can be synthesized, *e.g*., by conventional phosphodiester techniques and administered by, e.g., intravenous injection or infusion.

Methods for using antisense techniques for specifically inhibiting gene expression of genes whose sequence is known are well-known in the art (see, e.g., see US patents US5,981,732, US6,046,321, US6,107,091, US6,365,354, US6,410,323, US6,566,131 and US6,566,135).

"**Ribozymes**" refers to enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin- or hammerhead-motif ribozyme molecules that specifically and efficiently catalyze endonucleolytic cleavage of 5-HTR1_{D} mRNA sequences are thereby useful within the scope of the present invention. Specific ribozyme cleavage sites within any potential RNA target are initially identified by scanning the target molecule for ribozyme cleavage sites, which typically include the following sequences, GUA, GLTU and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, *e.g*., ribonuclease protection assays.

Both antisense oligonucleotides and ribozymes can be prepared by known methods. These include, without limitation, techniques for chemical synthesis such as, *e.g*., solid phase phosphoramadite chemical synthesis. Alternatively, asRNA molecules can be generated by *in vitro* or *in vivo* transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

"**RNAi**", as used herein, include, without limitation, small interfering RNAs (siRNAs), small hairpin RNAs (shRNAs) and microRNAs (miRNAs), targeted to a p16^{INK4} transcript; as well as RNAi vectors whose presence within a cell results in the production of an siRNA, shRNA or miRNA targeted to the target 5-p16^{INK4} transcript. Such siRNA, shRNA or miRNA comprises a portion of RNA that is complementary to a region of the target 5-p16^{INK4} transcript.

RNA interference is a multistep process and is generally activated by double-stranded RNA (dsRNA) that is homologous in sequence to the targeted p16^{INK4} gene. Introduction of long dsRNA into the cells of organisms leads to the sequence-specific degradation of homologous gene transcripts. The long dsRNA molecules are metabolized to small (e.g., 21-23 nucleotide) interfering RNAs (siRNAs, shRNAs or miRNAs) by the action of an endogenous ribonuclease known as Dicer. The interfering RNAs bind to a protein complex, termed RNA-induced silencing complex (RISC), which contains a helicase activity and an endonuclease activity. The helicase activity unwinds the two strands of RNA molecules, allowing the antisense strand to bind to the targeted p16^{INK4} RNA molecule. The endonuclease activity hydrolyzes the 5-HTR1_{D} RNA at the site where the antisense strand is bound. Therefore, RNAi is an antisense mechanism of action, as a single stranded (ssRNA) RNA molecule binds to the target p16^{INK4} RNA molecule and recruits a ribonuclease that degrades the p16^{INK4} RNA.

Methods for selecting an appropriate dsRNA or dsRNA-encoding vector are well-known in the art for genes whose sequence is known (see, *e.g.,* Tuschl et al., 1999. Genes Dev. 13(24):3191-7; Elbashir et al., 2001. Nature. 411(6836):494-8; Hannon, 2002. Nature. 418(6894) 244-51; McManus & Sharp, 2002. Nat Rev Genet. 3(10):737-47; McManus et al., 2002. RNA. 8(6):842-50; Brummelkamp et al., 2002. Science. 296(5567):550-3; US patents US6,573,099 and US6,506,559; and International patent applications WO1999032619, WO2001036646 and WO2001068836).

In a further aspect, the p16^{INK4a} inhibitor is a chemical entity that is chosen among the group comprising a peptidic ligand, an antagonist of p16^{INK4a} or a small compound molecule that is able to bind directly or allosterically to the p16^{INK4a} protein(s) reducing its activity or that is able to reduce the expression levels of the p16^{INK4a} mRNA(s) or protein(s) through defined or undefined mechanisms, also reducing its activity.

In a further aspect, the p16^{INK4a} inhibitor is a ligand, said ligand is an antibody, Fab, Fab', F(ab')2, Fv, dsFv, scFv, diabody, triabody, tetrabody or VHH domain.

"**Antibody**", as used herein, encompasses intact polyclonal antibodies, intact monoclonal antibodies, single-domain antibodies, nanobodies, antibody fragments (such as Fab, Fab', F(ab')2 and Fv fragments), single chain Fv (scFv) mutants, multispecific antibodies (such as bispecific antibodies generated from at least two intact antibodies), chimeric antibodies, humanized antibodies, human antibodies, fusion proteins comprising an antigen determination portion of an antibody, and any other modified immunoglobulin molecule comprising an antigen recognition site, so long as the antibodies exhibit the desired biological activity. An antibody can be of any the five major classes of immunoglobulins: IgA, IgD, IgE, IgG, and IgM, or subclasses (isotypes) thereof *(e.g.,* IgGl, IgG2, IgG3, IgG4, IgAl and IgA2), based on the identity of their heavy-chain constant domains referred to as α (alpha), δ (delta), ε (epsilon), γ (gamma) and µ (mu), respectively. The different classes of immunoglobulins have different and well-known subunit structures and three-dimensional configurations. Antibodies can be naked, or conjugated to other molecules such as toxins, radioisotopes, or any of the other specific molecules recited herein.

A "**monoclonal antibody**" refers to a homogeneous antibody population involved in the highly specific recognition and binding of a single antigenic determinant or epitope. This is in contrast to "**polyclonal antibodies**" that typically include different antibodies directed against different antigenic determinants. The term "monoclonal antibody" encompasses both intact and full-length monoclonal antibodies, as well as antibody fragments (such as Fab, Fab', F(ab')2, Fv), single chain (scFv) mutants, fusion proteins comprising an antibody portion, and any other modified immunoglobulin molecule comprising an antigen recognition site. Furthermore, "monoclonal antibody" refers to such antibodies made in any number of ways including, but not limited to, by hybridoma, phage selection, recombinant expression, and transgenic animals. The term "**humanized antibody**" refers to an antibody derived from a non-human (e.g., murine) immunoglobulin, which has been engineered to contain minimal non-human (e.g., murine) sequences. Typically, humanized antibodies are human immunoglobulins in which residues from the complementary determining region (CDR) are replaced by residues from the CDR of a non-human species (*e.g*., mouse, rat, rabbit, or hamster) that have the desired specificity, affinity, and capability. In some instances, the Fv framework region (FW) residues of a human immunoglobulin are replaced with the corresponding residues in an antibody from a non-human species that has the desired specificity, affinity and capability. Humanized antibodies can be further modified by the substitution of additional residues either in the Fv framework region and/or within the replaced non-human residues to refine and optimize antibody specificity, affinity, and/or capability. In general, humanized antibodies will comprise substantially all of at least one, and typically two or three, variable domains containing all or substantially all of the CDR regions that correspond to the non- human immunoglobulin whereas all or substantially all of the FR regions are those of a human immunoglobulin consensus sequence. Humanized antibody can also comprise at least a portion of an immunoglobulin constant region or domain (Fc), typically that of a human immunoglobulin.

In a still further aspect, the p16^{INK4a} inhibitor is a small compound molecule such a peroxisome proliferator-activated receptor γ (PPAR-γ) antagonist or such as a retinoid X receptor (RXR) antagonist. AMPK activators, SIRT1 activators or a combination thereof.

The retinoid X receptor (RXR) antagonists can be chosen among the following non-limitative list: UVI3003, however combined with a PPAR gamma antagonist, HX531, PA452, NEt-3IB, β-Apo-13-carotenone, LG100754, AGN1985393, Ro26-5405, LG101506, PA451, PA452, BI-1003, BI-1005, SR11179, UV13003, HX531, Danthron, Rhein, béta-apo-13-carrotene, R-etodolac, Sundilac sulfide, K-8003, K-8008, triptolide, TRC4, NSC-64358, Flvastatin, 9-cis retinoic acid, PA024, CD3254, LG100754, UVI3003, HX531 and all the components described in "Retinoid X Receptor Antagonists", Masaki Watanabe and Hiroki Kakuta, International Journal of Molecular Sciences 2018, 19, 2354, RXR antagonists based on the diazepinylbenzoic acid scaffold, (2E,4E,6Z)-7-[2-butoxy-3,5-bis(1,1-dimethylethyl)- phenyl]-3-methyl-2,4,6-octatrienoic acid, (2E,4E,)-(1 RS,2RS)-5-[2-(3,5-Di-tert-butyl-2-butoxy-phenyl)-cyclopropyl]-3-methyl-penta-2,4-dienoic acid, (2E,4E))-(1RS,2RS)-5-[2-(3,5-Di-tert-butyl-2-ethoxyphenyl)-cyclopropyl]-3-methyl-penta-2,4-dienoic acid, (2E,4E,6Z)-7-[3_{J}5-Bis(1,1-dimethylethyl)-2-ethoxy-phenyl]-3-methyl-2,4,6-octatrienoic acid ethyl ester, (2E,4E)-3-Methyl-5-[2-(2,6,6-trimethyl-cyclohex-1-enylethynyl)-cyclohept-1-enyl]-penta-2,4-dienoic acid, (2E,4E)-3-Methyl-5-[(1 RS,2RS)-2-(5,5,8)8-tetramethyl-3- propoxy-5,6,7,8-tetrahydronaphthalen-2-yl)- cyclopropyl]-penta-2,4-dienoic acid, (2E,4E,6Z)-3-Methyl-7-(5,5j8j8-tetramethyl-3-propoxy- 5,6,7,8 -tetrahydro-naphthalen-2-yl)-octa-2,4,6-trienoic, or 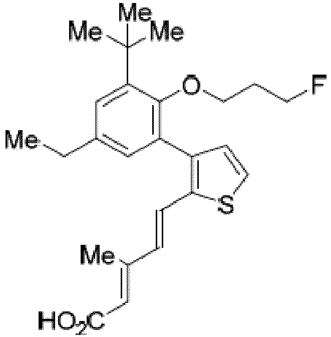 or 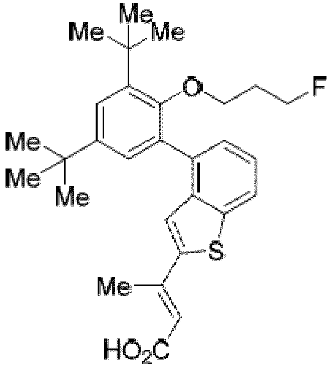 or 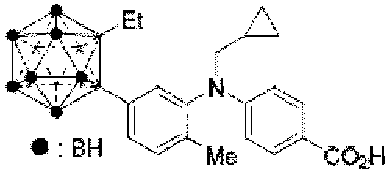 or 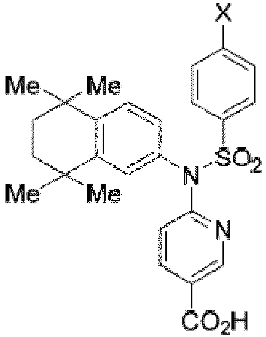 or 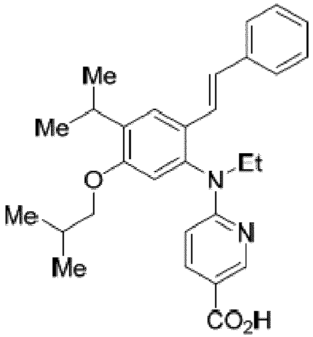 or 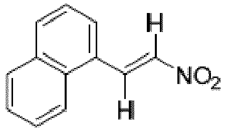 or 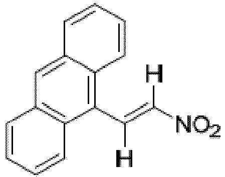 or 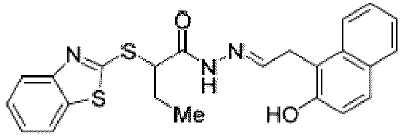 or 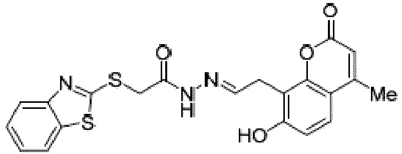 or 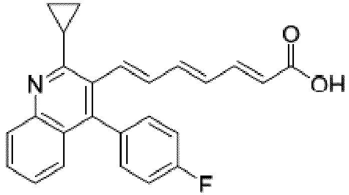 or 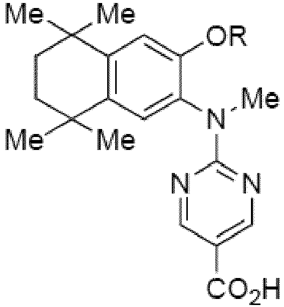 or 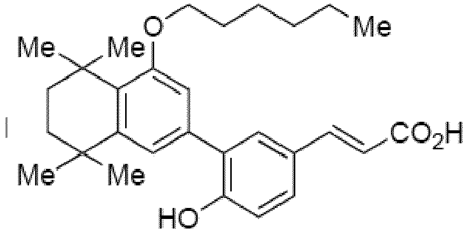 or 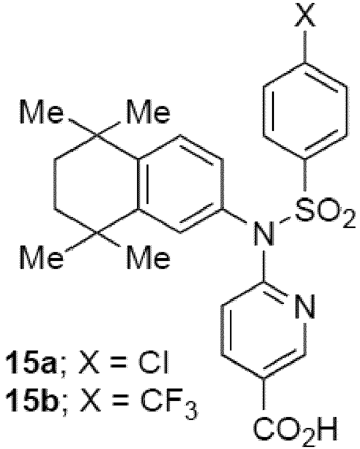 or 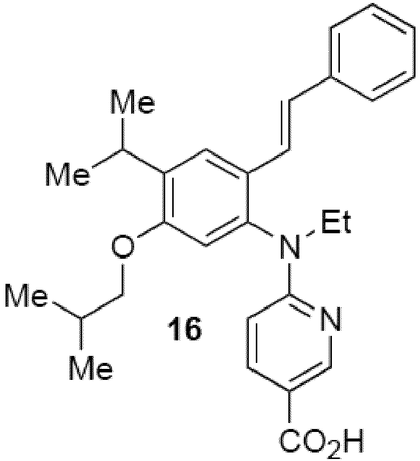 or 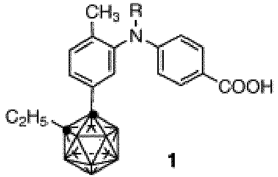 or peroxisome proliferator-activated receptors (PPARs; NR1C1-3), liver X receptors (LXRs; NR1H2-3), or farnesoid X receptor (FXR; NR1H4),

The PPAR-gamma or PPAR-alpha antagonists, in particular those that may cross the Brain barrier (screen patent databases) can be chosen among the following non-limitative list : Bisphenol A diglycidyl ether (BADGE), GW9662, isorhamnetin, T0070907, RUO 2-Chloro-5-nitrobenzanilide, 3-[[[2-Methoxy-4-(phenylamino)phenyl]amino]sulfonyl]-2-thiophenecarboxylic acid methyl ester, N-((2S)-2-(((1Z)-1-Methyl-3-oxo-3-(4-(trifluoromethyl)phenyl)prop-1-enyl)amino)-3-(4-(2-(5-methyl-2-phenyl-1,3-oxazol-4 yl)ethoxy)phenyl)propyl)propenamide, 2-Chloro-5-nitro-N-4-pyridinyl-benzamide,AZ6102, FH535, Fenofibric acid, GSK0660, GSK3787.
or 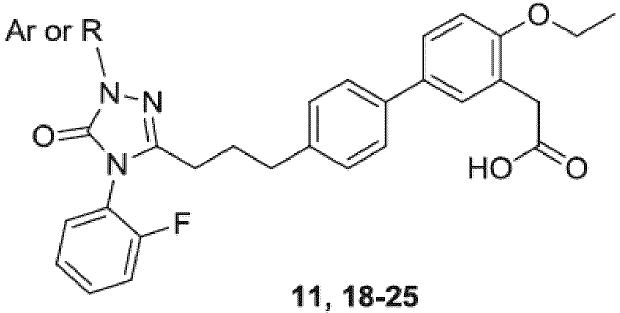 or 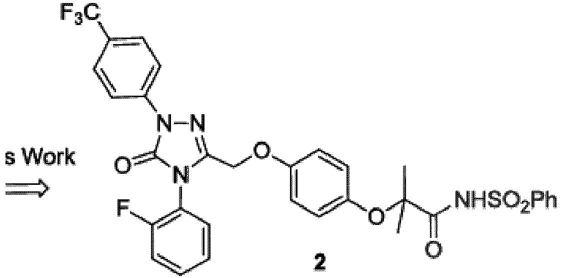 Or 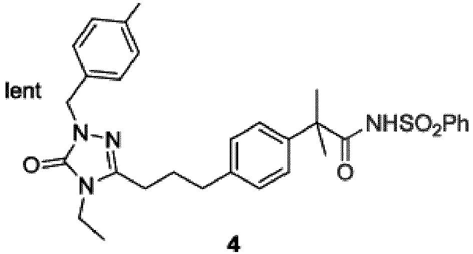 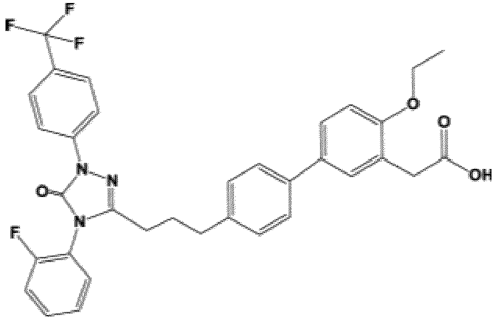 or

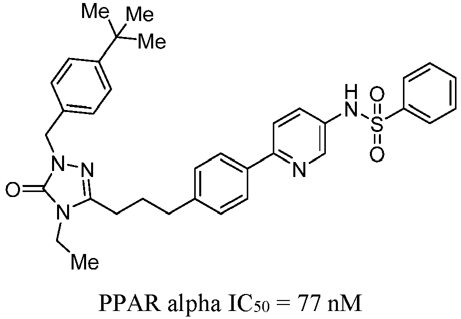

The small molecule SIRT1 activators can be chosen in the following non-limitative list: Nicotinamide (NAM), carba-NAD+ , thioacetyl-lysine peptides , and acetylated-lysine-ADP ribose conjugates, tenovins, MC2141, EX-527, resveratrol, all resveratrol analogs, quercetin and quercetin derivatives, fisetin, Alkylresorcinols, SRT1460, SRT1720, SRT2183 and (brain penetrant) SRT3025, STAC-5, butein, STAC-9, STAC-10.

The AMPK activators can be chosen in the following non-limitative list: Metformin, troglitazone, Pioglitazone, rosiglitazone, resveratrol, quercetin, genistein, epigallocatechin gallate, berberine, curcumin, ginsenside Rb1, alpha-lipoic acid, cryptotanshinone, AICAR, Thienopyridone, benzimidazole, salicyclate, , ex229 from patent application WO2010036613, Abbott A769662 compound and AICAR (5-amino-4-imidazolecarboxamide riboside), 991 [also known as ex229 from patent application WO2010036613), , ctivator-3, 2-[2-(4-(trifluoromethyl)phenylamino)thiazol-4-yl]acetic acid,, CNX-012-570, MK-8722, 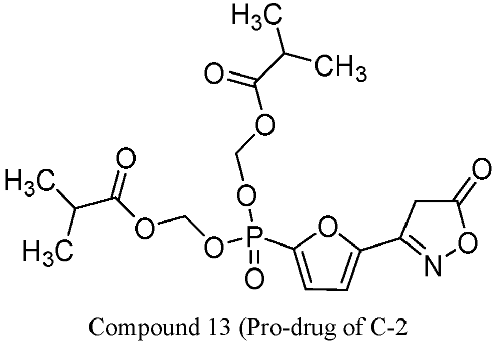 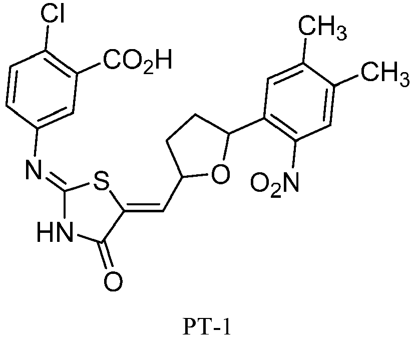 or 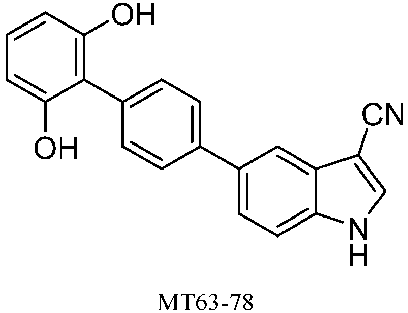 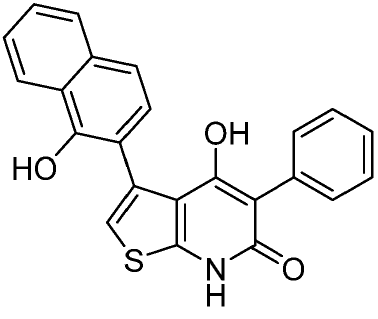 WO2009124636 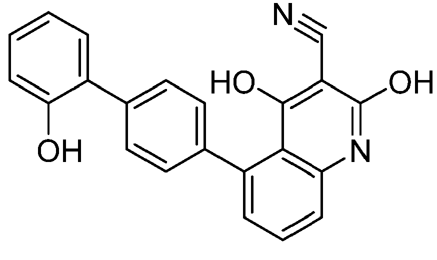 WO2009100130 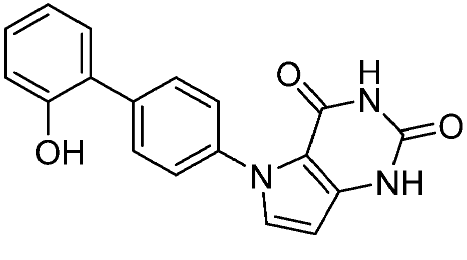 WO2011029855 WO201138307 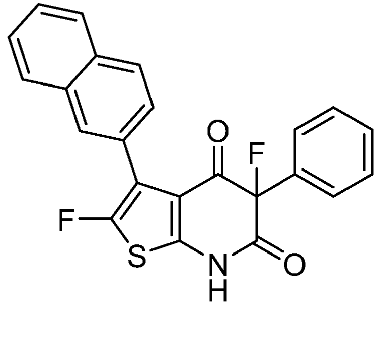 WO2011080277
or 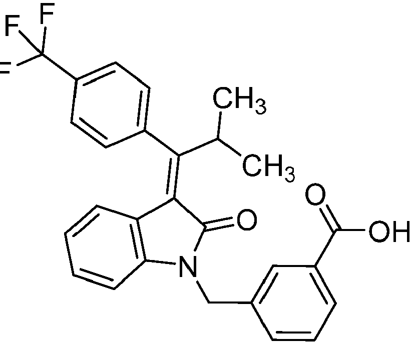 WO2011032320 WO2011033099

Compounds that stimulates the the activity of FOXO factors via but not only FOXO co-factors (e.g. β-catenin) or upstream regulators (e.g. AMPK, SIRT1), are also part of the compound or the invention they can be chosen from the non limitavie following list: resveratrol (Parket et al Nat Genet. 2005 Apr;37(4):349-50. Epub 2005 Mar 27.), and 3β-Methoxy-Pregnenolone (MAP4343), 17β-oestradiol (17ßE2), Lithium chloride, isoquercitrin (flavonoid with the resveratrol pharmacophore), and 11 flavonoids (compounds A to K) with the resveratrol pharmacophore (Farina et al., Sci Rep. 2017 Jun 21;7(1):4014. doi: 10.1038/s41598-017-04256-was shown below:

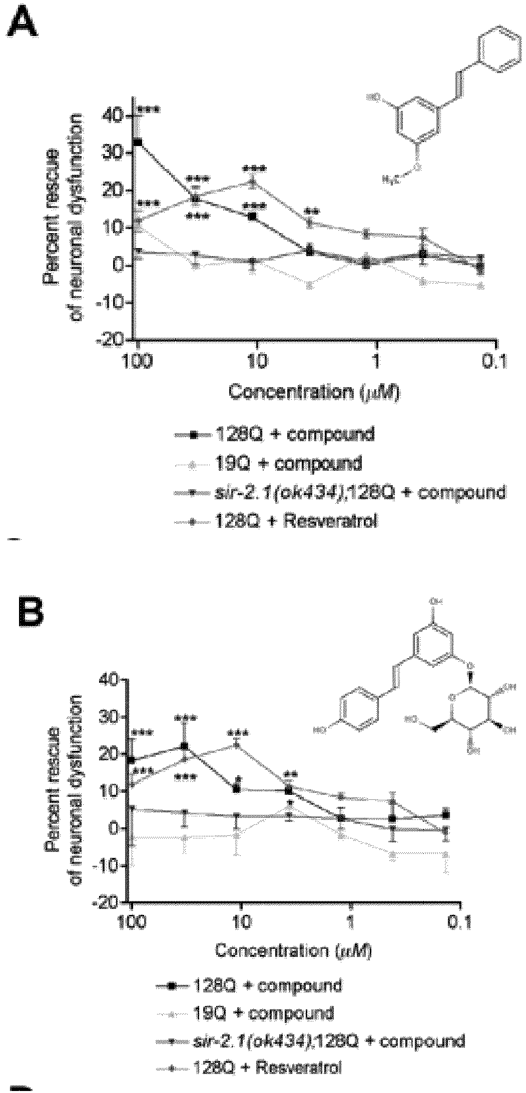

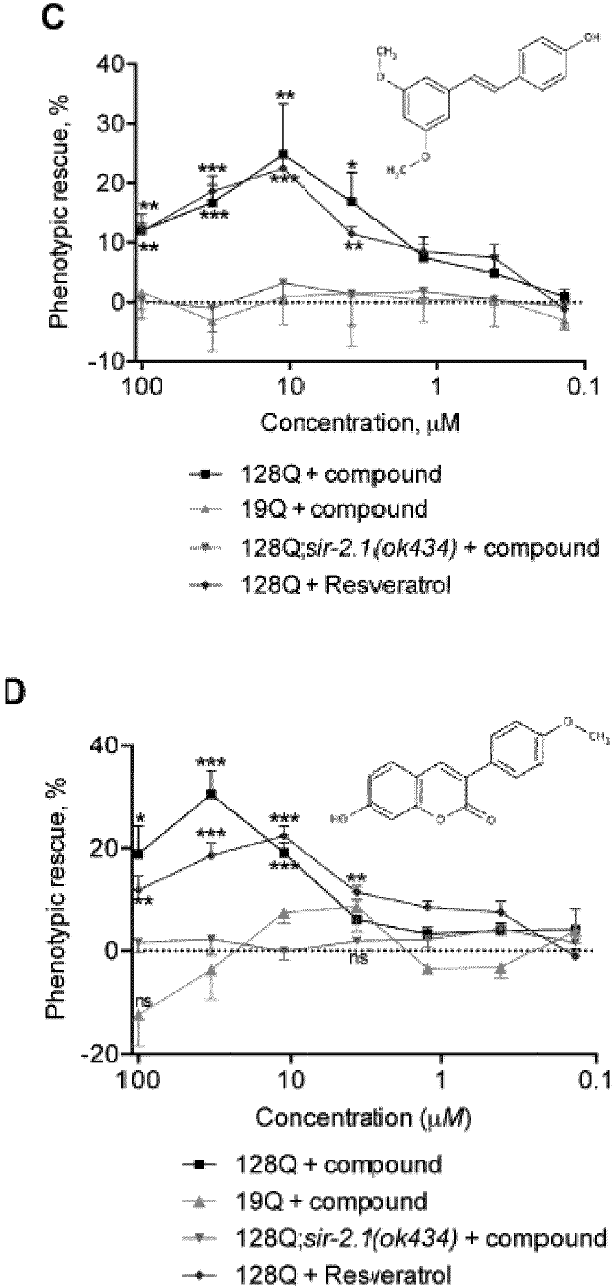

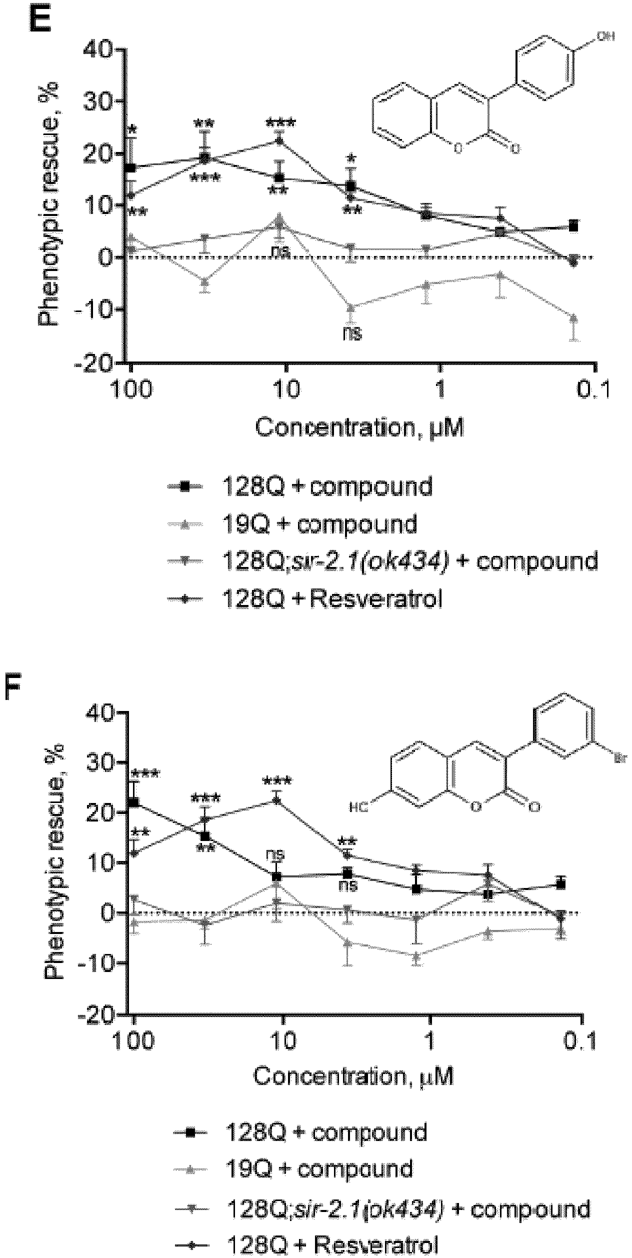

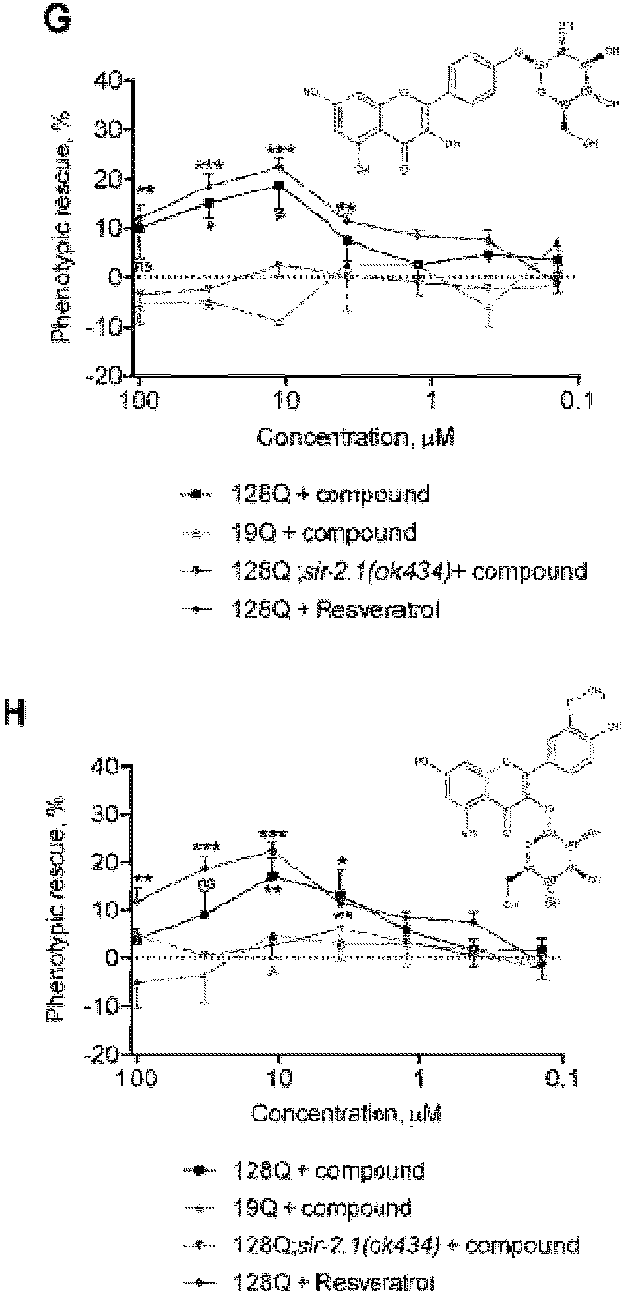

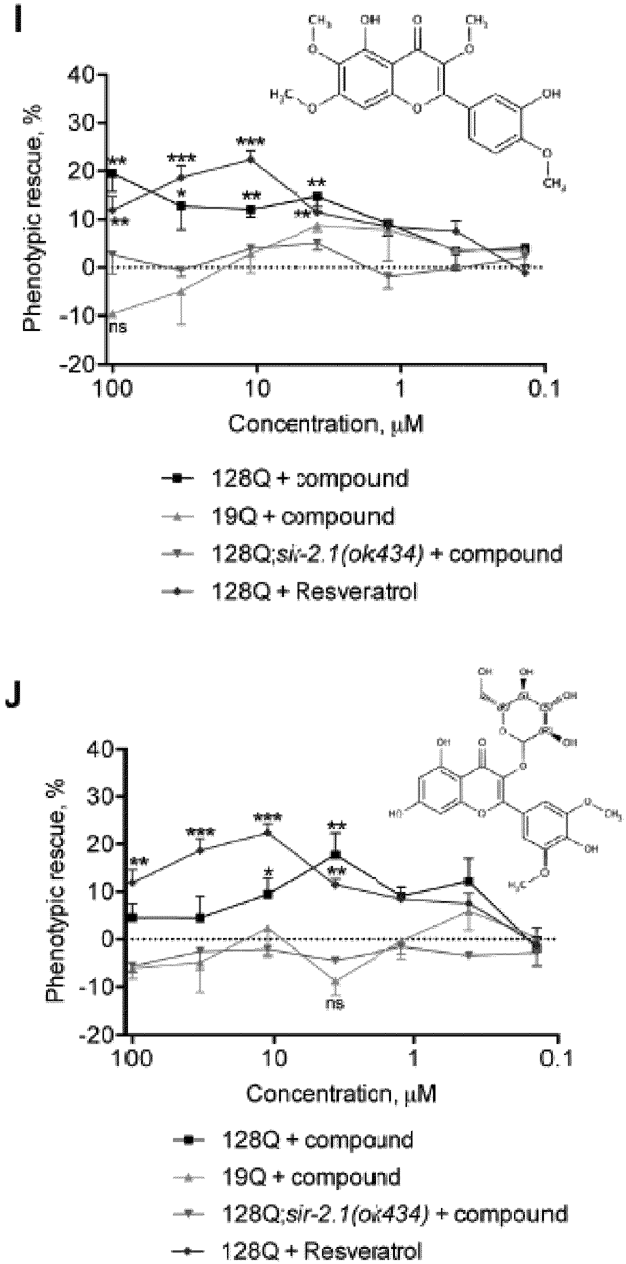

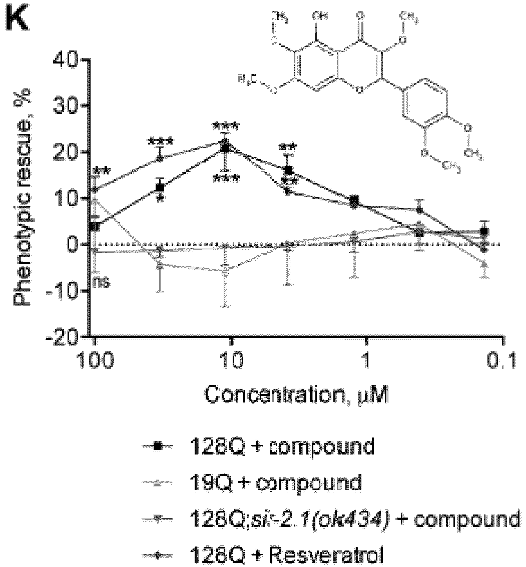

In a specific aspect the p16 ^{Ink4a} inhibitor according to the present invention is a polynucleotide for targeted gene editing. Examples of gene editing methods contemplated in the present invention include but are not limited to transcription activator-like effector nuclease (TALEN)-based editing system, clustered regularly interspaced short palindromic repeats-Cas9 SCRIR-Cas9), self-inactivating KamiCas9 system, meganucleases, zinc-finger nucleases (ZFNs) and the like (Nature. 2011 Nov 2; 479(7372): 232-236).

In a further aspect, the invention further relates to a composition comprising a p16^{INK4} inhibitor according to the present invention, for use in the prevention and/or treatment of HD.

The present invention also further relates to a pharmaceutical composition comprising at least one p16^{INK4} inhibitor according to the present invention, and at least one pharmaceutically acceptable excipient, for use in the prevention and/or treatment of HD.

As used herein, a "**pharmaceutically acceptable excipient**" refers to a non-toxic solid, semi-solid or liquid filler, diluent, encapsulating material or formulation auxiliary of any type. Pharmaceutically acceptable excipients that may be used in the compositions of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, silica, colloidal silica, magnesium trisilicate, polyvinyl pyrrolidone, cellulose-based substances (e.g., sodium carboxymethylcellulose), polyethylene glycol, polyacrylates, waxes, polyethylene-polyoxypropylene-block polymers, polyethylene glycol and wool fat.

The pharmaceutical composition according to the present invention may further comprise antioxidant agents, including, but not limited to, ascorbic acid, ascorbyl palmitate, BHT, potassium sorbate or *Rosmarinus officinalis* extracts.

The pharmaceutical composition according to the present invention may further comprise flavour agents, including, but not limited to, sugars, fruit or tea flavourings.

The pharmaceutical composition according to the present invention may further comprise pharmaceutically acceptable salts, including, but not limited to, acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The excipient can also be a solvent or dispersion medium containing, for example, water, ethanol, polyol (*e.g*., glycerol, propylene glycol, and liquid polyethylene glycol, and the like), suitable mixtures thereof, and vegetables oils such as oleic acid. The proper fluidity can be maintained, for example, by the use of a coating, such as lecithin (*i.e*., soy lecithin or de-greased soy lecithin), by the maintenance of the required particle size in the case of dispersion and by the use of surfactants.

The prevention of the action of microorganisms can be brought about by various antibacterial and antifungal agents, for example, parabens, chlorobutanol, phenol, sorbic acid, thimerosal, and the like.

In many cases, it will be preferable to include isotonic agents, for example, sugars or sodium chloride.

For a prolonged absorption of the pharmaceutical composition according to the present invention, agents delaying absorption can be added, including, but not limited to, aluminium monostearate and gelatine.

In another aspect, the composition of the invention further contains a nucleic acid sequence encoding a peptide or polypeptide for cell-specific targeting and/or contains a nucleic acid enabling a cell-specific expression.

In another aspect, the composition or the pharmaceutical composition of the invention further comprises one or more active agent(s) for treating HD and/or one or more agent(s) for treating side effects of said active agent(s).

The present invention further relates to a medicament comprising a p16^{INK4} inhibitor according to the present invention, for use in the prevention and/or treatment of HD.

In one embodiment of the invention, the p16^{INK4} inhibitor, the composition, the pharmaceutical composition comprising the p16^{INK4} inhibitor or is used in a therapeutically effective amount.

In one embodiment, the p16^{INK4} inhibitor, the composition, the pharmaceutical composition or the medicament according to the present invention is to be administered at a dose determined by the skilled artisan and personally adapted to each subject.

It will be understood that the total daily usage of the p16^{INK4} inhibitor, the composition, the pharmaceutical composition or the medicament according to the present invention will be decided by the attending physician within the scope of sound medical judgment. The specific therapeutically effective amount for any particular subject will depend upon a variety of factors including the condition being treated and the severity of the condition; the specific composition employed, the age, body weight, general health, sex and diet of the subject; the time of administration, route of administration, the duration of the treatment; drugs used in combination or coincidental with the p16^{INK4} inhibitor, the composition, the pharmaceutical composition or the medicament according to the present invention; and like factors well known in the medical arts. For example, it is well within the skill of the art to start doses of a therapeutic compound at levels lower than those required to achieve the desired therapeutic effect and to gradually increase the dosage until the desired effect is achieved; but, at the opposite, it can be equally useful to start with a loading dose, a manner to reach steady-state plasma concentration more quickly, and then to follow with a maintenance dose calculated to exactly compensate the effect of the elimination process.

In one embodiment, a therapeutically effective amount of the p16^{INK4} inhibitor, the composition, the pharmaceutical composition or the medicament according to the present invention is to be administered at least once a day, at least twice a day, at least three times a day.

In one embodiment, a therapeutically effective amount of the p16^{INK4} inhibitor, the composition, the pharmaceutical composition or the medicament according to the present invention is to be administered every two, three, four, five, six days.

In one embodiment, a therapeutically effective amount of the p16^{INK4} inhibitor, the composition, the pharmaceutical composition or the medicament according to the present invention is to be administered twice a week, every week, every two weeks, every three weeks, once a month.

In one embodiment, a therapeutically effective amount of the p16^{INK4} inhibitor, the composition, the pharmaceutical composition or the medicament according to the present invention is to be administered every month, every two months, every three months, every four months, every five months, every six months, once a year.

In one embodiment, a therapeutically effective amount of the 5 p16^{INK4} inhibitor, the composition, the pharmaceutical composition or the medicament according to the present invention is to be administered for a period of time of about one day, two days, three days, four days, five days, six days, a week, two weeks, three weeks, a month, two months, three months, six months, a year, or over longer periods such as, e.g., for several years or for the rest of the life of the subject.

In one embodiment, the p16^{INK4} inhibitor, the composition, the pharmaceutical composition or the medicament according to the present invention is to be administered systemically or locally.

In one embodiment, the p16^{INK4} inhibitor, the composition, the pharmaceutical composition or the medicament according to the present invention is to be administered orally, buccally, by injection, by percutaneous administration, parenterally, intraperitoneal, by endoscopy, topically, transdermally, transmucosally, nasally, by inhalation spray, rectally, vaginally, intratracheally, or via an implanted reservoir.

In one embodiment, the p16^{INK4} inhibitor, the composition, the pharmaceutical composition or the medicament according to the present invention is to be orally administered.

Examples of formulations adapted to oral administration include, but are not limited to, solid forms, liquid forms and gels.

Examples of solid forms adapted to oral administration include, but are not limited to, pill, tablet, capsule, soft gelatin capsule, hard gelatin capsule, dragees, granules, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet.

Examples of liquid form adapted to oral administration include, but are not limited to, solutions, suspensions, drinkable solutions, elixirs, sealed phial, potion, drench, syrup, liquor and sprays.

In one embodiment, the p16^{INK4} inhibitor, the composition, the pharmaceutical composition or the medicament according to the present invention is to be injected, preferably systemically injected.

Examples of formulations adapted to systemic injections include, but are not limited to, liquid solutions or suspensions, solid forms suitable for solution in, or suspension in, liquid prior to injection.

Examples of systemic injections include, but are not limited to, intravenous, intracranial, intralymphatic, intraperitoneal, intramuscular, subcutaneous, intradermal, intraarticular, intrasynovial, intrasternal, intrathecal, intravesical, intrahepatic, intralesional, intracavernous, infusion techniques and perfusion.

In another embodiment, when injected, the p16^{INK4} inhibitor, the composition, the pharmaceutical composition or the medicament according to the present invention is sterile. Methods for obtaining a sterile composition, pharmaceutical composition, medicament or nutraceutical composition include, but are not limited to, GMP synthesis (GMP stands for "Good manufacturing practice").

### Delivery systems

In one embodiment, the composition, pharmaceutical composition or medicament according to the present invention may be used in conjunction with delivery systems that facilitate delivery of the agents to the central nervous system. For example, various blood brain barrier (BBB) permeability enhancers may be used to transiently and reversibly increase the permeability of the blood brain barrier to a treatment agent. Such BBB permeability enhancers include, but are not limited to, leukotrienes, bradykinin agonists, histamine, tight junction disruptors (*e.g.*, zonulin, zot), hyperosmotic solutions (*e.g*., mannitol), cytoskeletal contracting agents, and short chain alkylglycerols (*e.g*., 1-O-pentylglycerol). Oral, sublingual, parenteral, implantation, nasal and inhalational routes can provide delivery of the active agent to the central nervous system. In some embodiments, the p16^{INK4}inhibitor, the composition, the pharmaceutical composition or the medicament according to the present invention may be administered to the central nervous system with minimal effects on the peripheral nervous system.

In another embodiment, the p16^{INK4} inhibitor, the composition, the pharmaceutical composition or the medicament according to the present invention is delivered in the form of exosomes, nanoparticulate, or polymeric nanoparticles drug delivery system.

### Sustained-release

In one embodiment, the at least one p16^{INK4} inhibitor, the composition, the pharmaceutical composition or the medicament according to the present invention is to be administered in an immediate release form.

In one embodiment, the p16^{INK4} inhibitor, the composition, the pharmaceutical composition or the medicament according to the present invention is to be administered in a mixed-release form.

In one embodiment, the p16^{INK4} inhibitor, the composition, the pharmaceutical composition or the medicament according to the present invention is to be administered in an enterically-coated form.

In one embodiment, the p16^{INK4} inhibitor, the composition, the pharmaceutical composition or the medicament according to the present invention is to be administered in a sustained-release form.

In one embodiment, the p16^{INK4} inhibitor, the composition, the pharmaceutical composition or the medicament according to the present invention comprises a delivery system that controls the release of the active ingredients.

In one embodiment, the subject is an animal, preferably a mammal, more preferably a primate, even more preferably a human.

In one embodiment, the subject is a male. In one embodiment, the subject is a male or a female.

In one embodiment, the subject is an adult.

In one embodiment, the subject is a child.

In one embodiment, the subject is a teenager.

In one embodiment, the subject is over 10, 15, 18 or 20 years old. In one embodiment, the subject is over 30 years old. In one embodiment, the subject is over 40 years old.

In one embodiment, the subject is over 50 years old. In one embodiment, the subject is over 55 years old. In one embodiment, the subject is over 60 years old.

In one embodiment, the subject is/was diagnosed with HD, presents/presented a genetic predisposition to HD or is affected, preferably diagnosed with HD. In one embodiment, the subject already received a HD treatment.

### Prevention and Treatment

The present invention further relates to a p16^{INK4} inhibitor, a composition, a pharmaceutical composition or the medicament, for use in preventing and/or treating of HD in a subject in need thereof. It also relates to methods of preventing and/or treating HD, by administering to a subject in need thereof the p16^{INK4} inhibitor, the composition, the pharmaceutical composition, the medicament or the vaccine composition according to the present invention.

Those in need of treatment include those already with the disorder as well as those prone to have the disorder or those in whom the disorder is to be prevented. A subject or mammal is successfully "treated" if, after receiving a therapeutic amount of the inhibitor or composition according to the present invention, the patient shows one or more of the following observable and/or measurable changes: amelioration related to one or more of the symptoms associated with the specific disease or condition, reduction of morbidity and mortality and improvement in quality of life issues. Parameters for assessing successful treatment and improvement in the disease are readily measurable by routine procedures familiar to a physician such as for example but not only the United Huntington's Disease Rating Scale (UHDRS) and other scales that are being developed such as for example but not only the Huntington's disease Cognitive Assessment Battery (HD-CAB).

The present invention further relates to a method of preventing or treating HD in a subject in need thereof comprising the administration of a p16^{INK4} inhibitor, a composition, a pharmaceutical composition or the medicament comprising said p16^{INK4} inhibitor.

The present invention also relates to a method of reducing mortality of neurons and/or NSCs in a HD subject in need thereof comprising the administration of a p16^{INK4} inhibitor.

The present invention also relates to a method for protecting HD neurons and/or HD NSCs from chronic and/or maladaptive senescence responses comprising the administration of a p16^{INK4} inhibitor.

The present invention further relates to a method for reducing DNA Damage Repair persistence in a HD subject in need thereof comprising the administration of a p16^{INK4} inhibitor.

The present invention also relates to a method for restoring a normal activity in HD neurons comprising the administration of a p16^{INK4} inhibitor.

Prevention is successful if, after receiving a therapeutic amount of the inhibitor or composition according to the present invention, the patient does not present early signs and symptoms or a delayed progression of the disease.

### BRIEF DESCRIPTION OF THE DRAWINGS

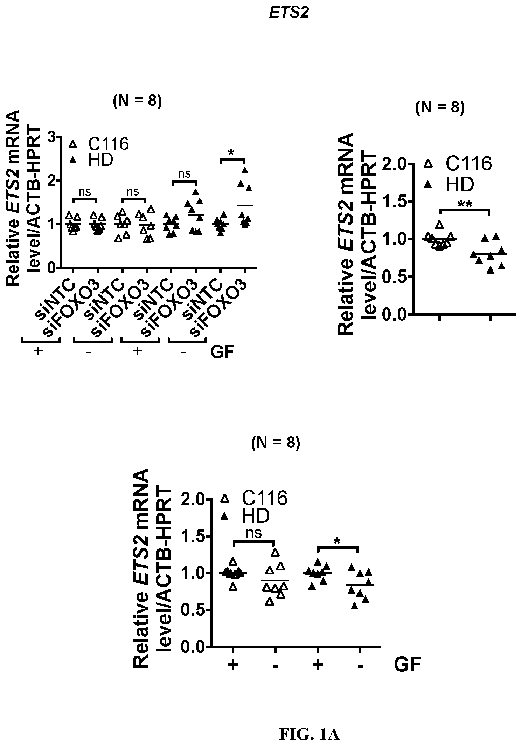

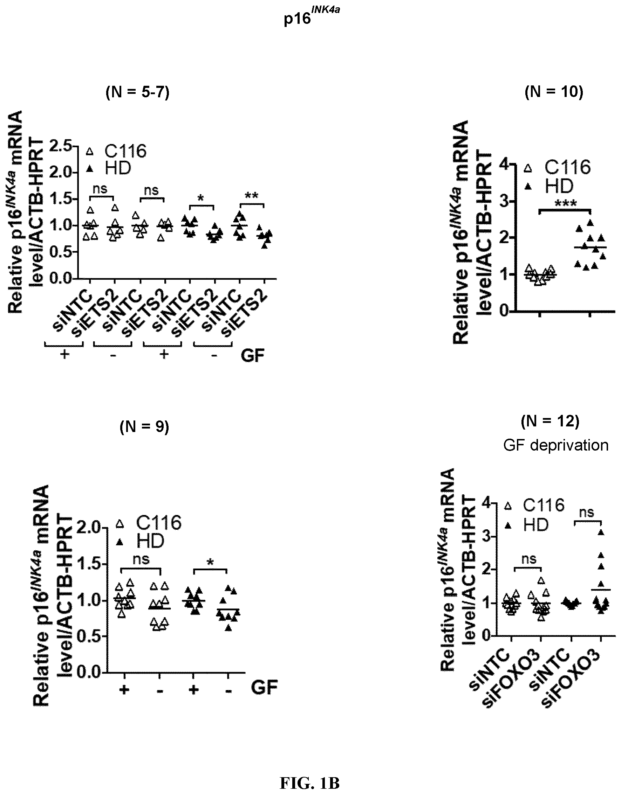

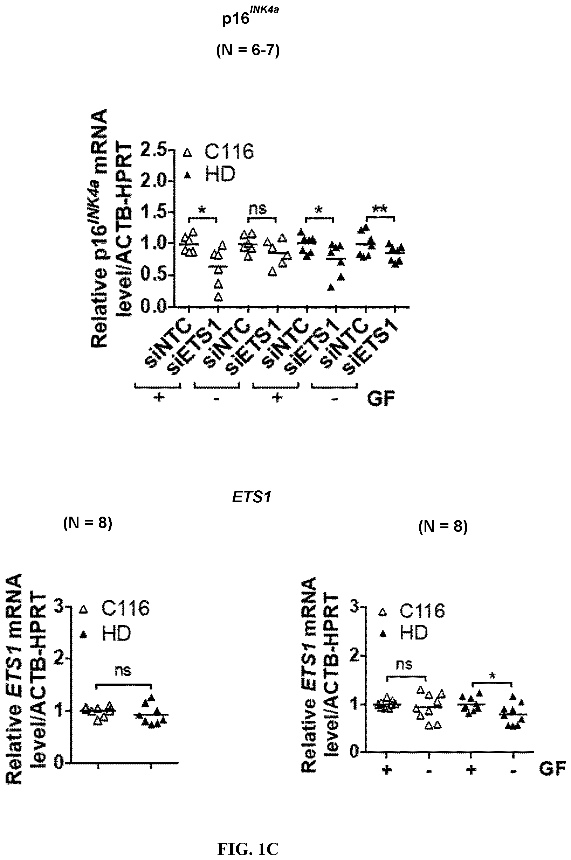

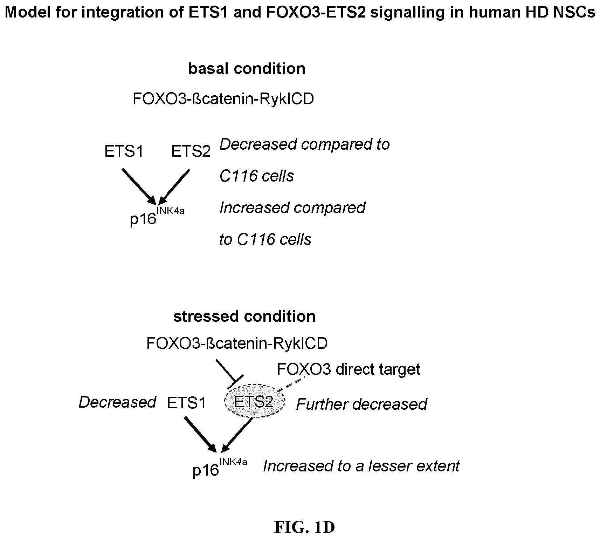

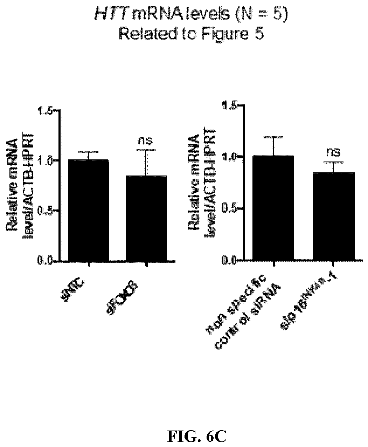

**Figure 1** relates to the characterization of FOXO3-ETS2, ETS2-p16^{INK4a} and ETS1-p16^{INK4a} interactions on gene regulation levels in human HD and normal-HTT NSCs.
   In experiments involving siRNA treatments, mRNA levels are normalized to cells treated with non-targeting control (NTC) siRNAs. In the other experiments, mRNA levels are normalized to C116 cells or cells without growth factor (GF) deprivation. ns, not significant.
   **(A)** *ETS2* mRNA levels are increased by *FOXO3* reduction in HD NSCs subjected to GF deprivation with no effect observed in basal conditions nor in normal *HTT* cells (left panel: ^{∗}*P* < 0.05). *ETS2* mRNA levels are decreased in HD NSCs (middle panel: ^{∗∗}*P* < 0.01). GF deprivation does not change *ETS2* mRNA levels in C116 NSCs and decrease *ETS2* mRNA levels in HD NSCs (right panel: **P* < 0.05).
   **(B)** *p16^{INK4a}* mRNA levels are decreased by *ETS2* reduction in HD NSCs in basal conditions and in cells subjected to stress with no effect detected in normal *HTT* cells (left panel: ^{∗}*P* < 0.05, ^{∗∗}*P* < 0.01). *p16^{INK4a}* mRNA levels are increased in HD NSCs (middle left panel: ****P* < 0.001). GF deprivation does not change p16^{INK4a} mRNA levels in C116 NSCs and decrease *p16^{INK4a}* mRNA levels in HD NSCs (middle right panel: **P* < 0.05). *p16^{INK4a}* mRNA levels tend to be increased by FOXO3 knockdown in HD NSCs subjected to GF deprivation (right panel: not significant with *P* = 0.0736).
   **(C)** *p16^{INK4a}* mRNA levels are decreased by *ETS1* reduction in C116 NSCs in basal conditions and in HD NSCs in both basal and stress conditions (left panel: ^{∗}*P* < 0.05, ^{∗∗}*P* < 0.01). *ETS1* mRNA levels are unchanged in HD compared to C116 NSCs (middle panel). GF deprivation does not change *ETS1* mRNA levels in C116 NSCs and decreases *ETS1* mRNA levels in HD NSCs (right panel: **P* < 0.05). **(D)** Working model for effect of FOXO3 target reprogramming on the ETS2-p16^{INK4a} pathway.

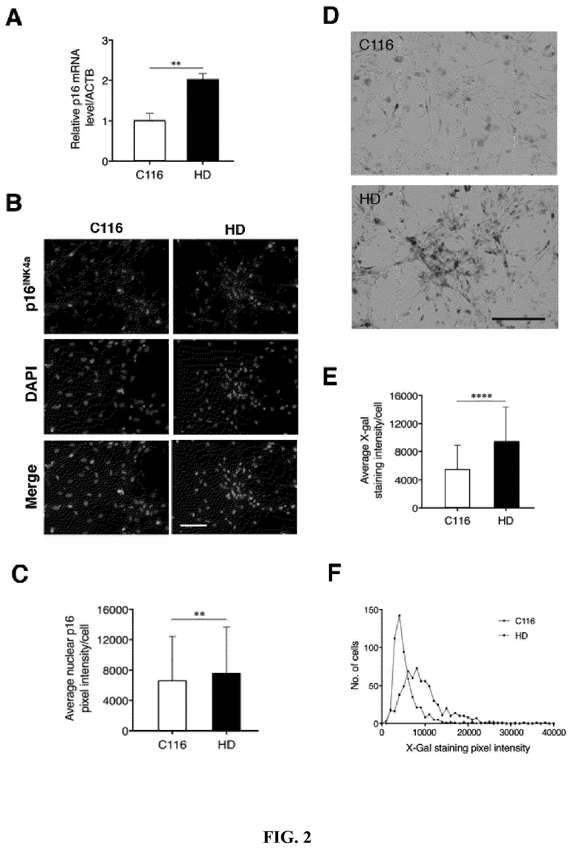

**Figure 2** describes increased levels of p16^{INK4a} and exhibit senescent phenotype characterized by increased SA-β-gal activity in human HD prepatterned NSCs .
   **(A)** The *p16^{INK4a}* mRNA levels are increased in HD prepatterned NSCs. Data are mean±SD (***P* < 0.01), N = 3.
   **(B)** Representative images for modest p16^{INK4a} increase in HD NSCs. Scale bar in all panels: 100 µm.
   **(C)** Quantification of nuclear p16^{INK4a} pixel intensity for N = 532 C116 NSCs and N = 1000 HD NSCs. Data are mean±SD (***P* < 0.01).
   **(D)** Representative images for increased expression of SA-β-gal activity in HD NSCs. Scale bar in all panels: 200 µm.
   **(E)** Quantification of SA-β-gal activity for N = 547 C116 NSCs and N = 645 HD NSCs. Data are mean±SD (*****P* < 0.0001).
   **(F)** Frequency distribution of SA-β-gal signals for data shown in panel E.

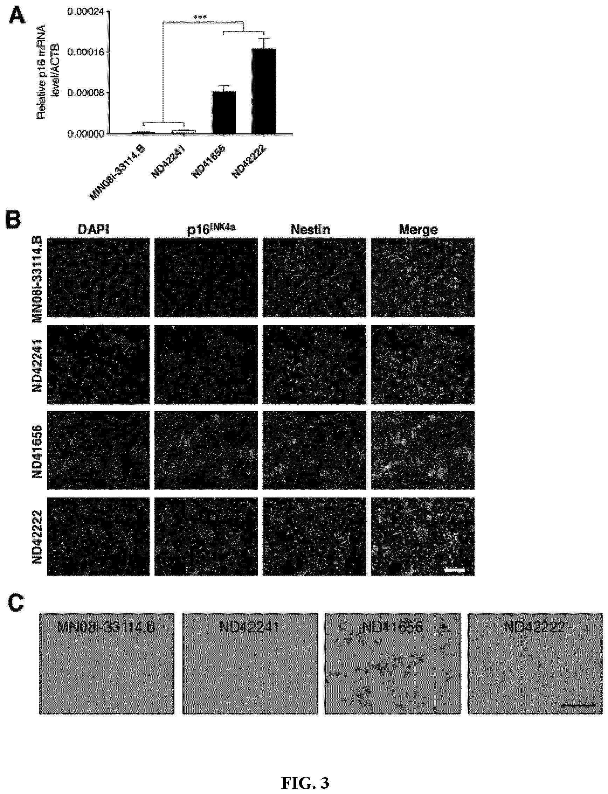

**Figure 3** shows that increased levels of p16^{INK4a} and elevated SA-β-gal activity are also characteristic of other non-isogenic HD NSC lines.
   **(A)** p16^{INK4a} mRNA levels as determined by RT-PCR analysis in control NSCs (blue bars; MN08i-33114.B and ND42241) and HD NSCs (red bars; ND41656 - CAG 57 and ND42222 - CAG 109). Data are mean ± SD (N = 3). ****P* < 0.001 (one-way ANOVA; Tukey's multiple comparison test).
   **(B)** Immunofluorescence analysis reveals robust increase of p16^{INK4a} in HD NSCs. Scale bar in all panels: 100 µm.
   **(C)** Representative images showing increased expression of SA-β-gal activity in HD NSCs (ND41656 - CAG 57; ND42222 - CAG 109) compared to control NSCs (MN08i-33114.B and ND42241). 10X magnification. Scale bar in all panels: 200 µm.

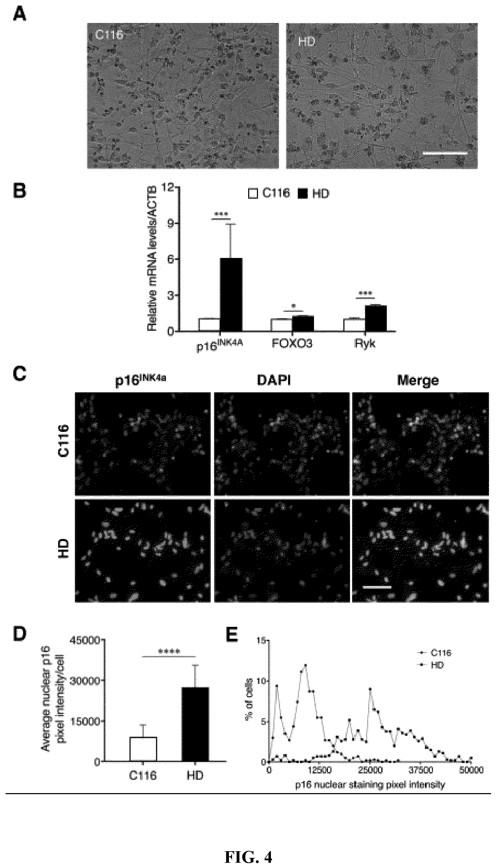

**Figure 4** shows that p16^{INK4a} expression is elevated in human HD MSNs.
   **(A)** Representative images of human MSNs prepared from NSCs using defined enhanced media (Synaptojuice medium).
   **(B)** RT-PCR analysis of p16^{INK4a}, FOXO3, and Ryk in C116 and HD MSNs reveals modest increase of FOXO3 mRNA levels and robust increase of p16^{INK4a} and Ryk mRNA levels in HD MSNs. Data are mean±SD (^{∗}*P* < 0.05, ^{∗∗∗}*P* < 0.001). N = 3.
   **(C)** Immunofluorescence analysis reveals dramatic increase of p16^{INK4a} in HD MSNs. Scale bar in all panels: 100 *µ*m .
   **(D)** Quantification of nuclear p16^{INK4a} pixel intensity for N = 596 C116 NSCs and N = 609 HD NSCs. Data are mean ± SD (*****P* < 0.0001).
   **(E)** Frequency distribution of nuclear p16^{INK4a} signals for data shown in Panel D.

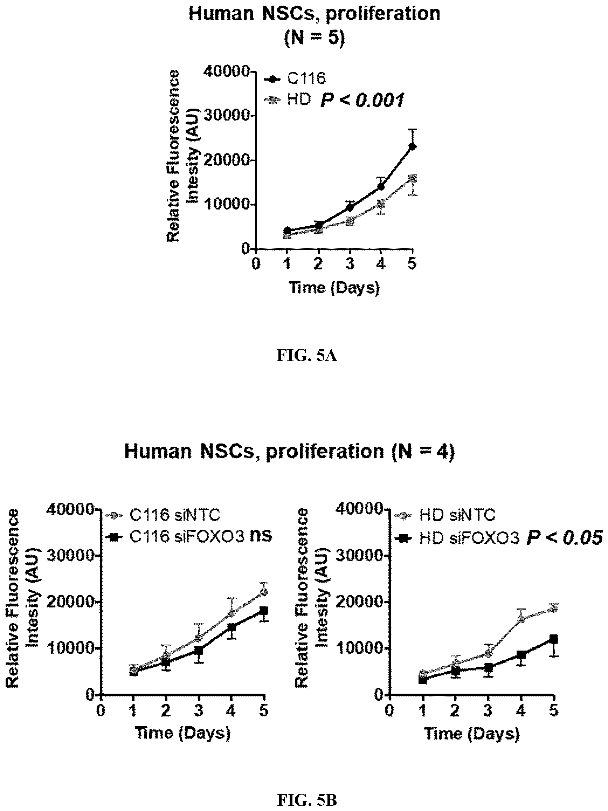

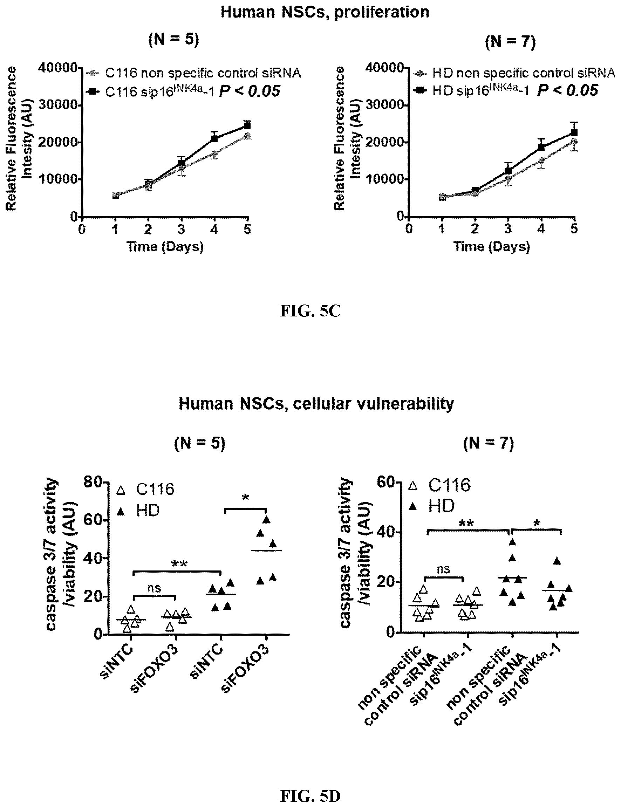

**Figure 5** shows that FOXO3 and p16^{INK4a} oppositely modulates the vulnerability of human HD neural stem cells.
   In cell growth assays, significance was tested using two-way ANOVA. In cellular vunerability assays, significance was tested using Student's t-test. ns: not significant.
   **(A)** Human HD NSCs show reduced rates of cell growth. Data are mean±SEM.
   **(B)** Reducing *FOXO3* does not alter the growth of C116 NSCs (left panel). Reducing *FOXO3* strongly reduces the growth of human HD NSCs (right panel), with no change detected in *HTT* mRNA levels (see Fig 6C, left panel). Data are mean±SEM.
   **(C)** Reducing *p16^{INK4a}* slightly increases the growth of C116 (left panel) and HD (right panel) NSCs. Reducing *p16^{INK4a}* does not alter *HTT* mRNA levels in HD NSCs (see Fig 6C, right panel). Data are mean±SEM.
   **(D)** Reducing *FOXO3* increases the mortality of human HD NSCs with no effect detected in C116 NSCs (left panel: ^{∗}*P* < 0.05, ^{∗∗}*P* < 0.01). Reducing *p16^{INK4a}* decreases the mortality of human HD NSCs with no effect detected in C116 NSCs (right panel: **P* < 0.05).

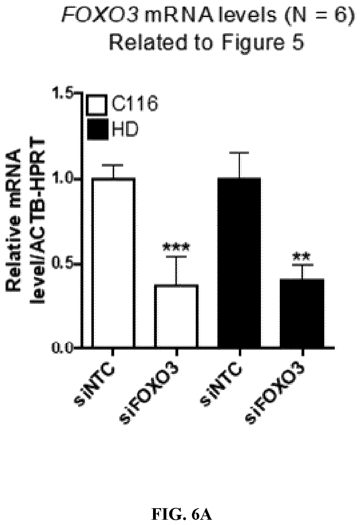

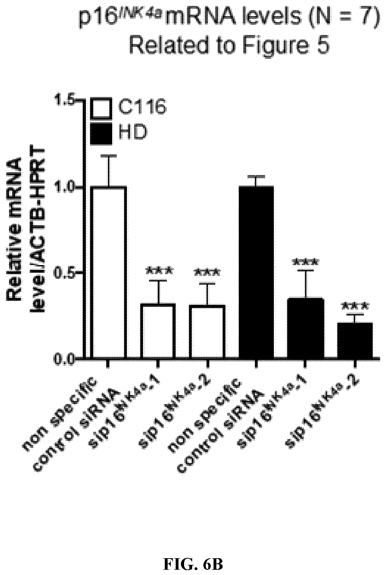

**Figure 6** presents gene target expression levels upon treatment with siRNAs and *HTT* expression levels upon reduction of *FOXO3* or reduction of *p16^{INK4a}.*
   Data are mean±SD. **(A)** *FOXO3* mRNA levels are decreased by siRNA treatment HD and normal *HTT* NSCs*.* ^{∗∗}*P* < 0.01 and ^{∗∗∗}*P* < 0.001 compared to NTC siRNAs. Related to Fig 5. (B)*p16^{INK4a}* mRNA levels are decreased by siRNA treatment HD and normal *HTT* NSCs. ^{∗∗∗}*P* < 0.001 compared to non-specific control siRNA treatment. Related to Fig 5. (C) *HTT* mRNA levels are unchanged by FOXO3 or p16^{INK4a} siRNA treatment in HD NSCs. ns, not significant.

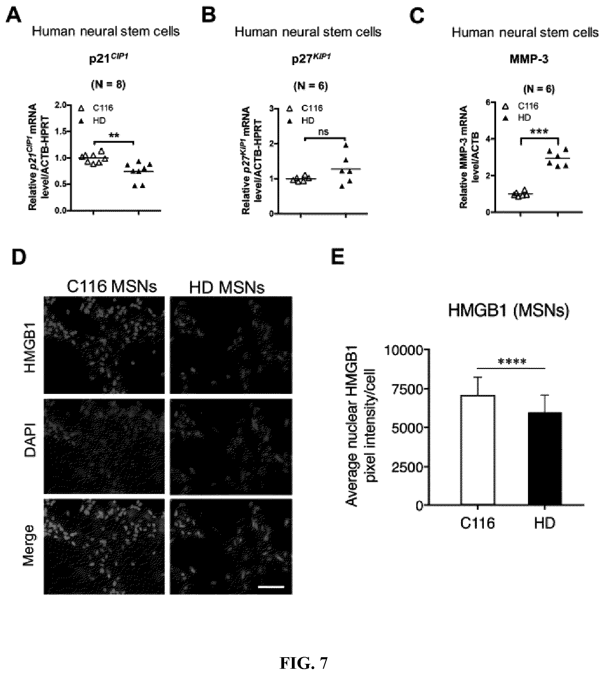

**Figure 7** presents relevant markers of senescence evaluated in HD NSCs and MSNs.
   **(A)** *p21^{CIP1}* mRNA levels are decreased in HD compared to C116 NSCs (^{∗∗}*P* < 0.01). **(B)** *p27^{KIP1}* mRNA levels are unchanged in HD compared to C116 NSCs. ns, not significant. **(C)** *MMP-3* mRNA levels are increased in HD compared to C116 NSCs (****P* < 0.001). **(D)** Immunofluorescence analysis reveals depletion of HMGB1 from nuclei of HD MSNs compared to C116 MSNs. Scale Bar in all panels: 100 µm. **(E)** Quantification of nuclear HMGB1 pixel intensity for N = 437 C116 NSCs and N = 491 HD NSCs. Data are mean±SD (****P < 0.0001).

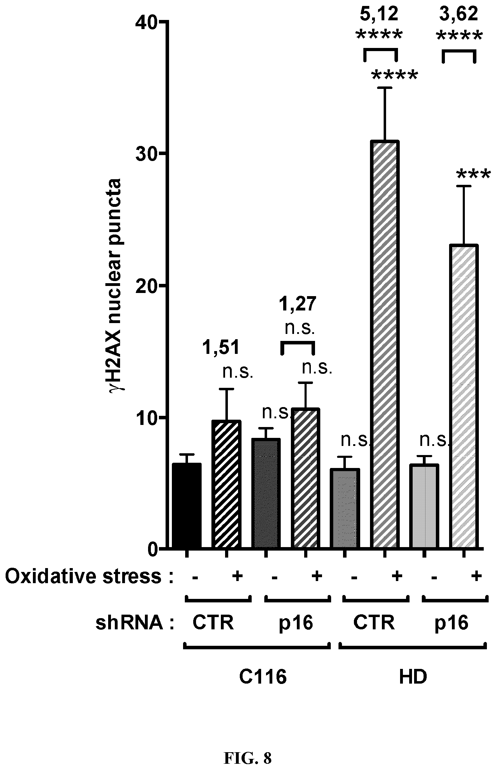

**Figure 8**: represents the expression of γH2AX, a marker of the DNA Damage Repair (DDR) machinery, in response to oxidative stress in HD and control (C116) NSCs stably expressing non-targeting shRNA (CTR) or shRNA directed against p16^{INK4a} (p16). HD and control (C116) NSCs stably expressing non-targeting shRNA (CTR) or shRNA directed against p16^{INK4a} (p16) were either treated with 400 mM H2O2 for 1 hour or left untreated (oxidative stress is indicated below the axis). Treated cells C116 and HD were then washed and put back in culture for 24 hours. All cells were then processed for immunofluorescence for γH2AX. Nuclear γH2AX puncta were then counted and plotted. Bold numbers indicate the fold change of nuclear γH2AX puncta over untreated cells. T-test was used for statistics. ***p<0.001; ****p<0.0001; n.s.: not significant. Data are mean±SD for a total of 45-87 cells.

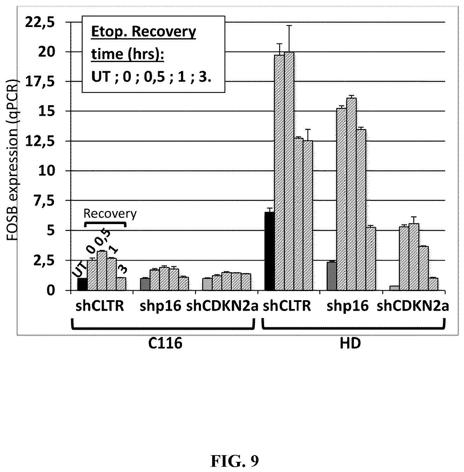

**Figure 9** represents the expression of FOSB (qPCR) in MSNs differentiated from HD and control (C1116) NSCs stably expressing non-targeting shRNA (CLTR), shRNA directed against p16^{INK4a} (shp16) or shRNA targeting the expression of both p16^{INK4a} and p14^{ARF} (shCDKN2a). 5 µM Etoposide (Etop.) was applied to MSNs differentiated from NSCs stably expressing the indicated shRNA for 1 hour, except for untreated (UT, solid bars) samples. Cells were then washed and put back in culture for the indicated recovery times (dashed bars, increasing recovery times from left to right as indicated: from left to right: 0-hour, 0.5-hour, 1 hour, 3 hours). All samples were then processed for RT-qPCR and FOSB expression was normalized to the expression of the housekeeping gene HPRT. Data are mean±SD.

### EXAMPLES

The present invention is further illustrated by the following examples.

### Example 1: Materials and Methods

### Cell Culture

We used human iPSCs derived from an HD patient (female - 20 years old: 72Q/19Q) and their CAG-corrected counterpart (21Q/19Q: C116) [An MCet al. Genetic correction of Huntington's disease phenotypes in induced pluripotent stem cells. Cell Stem Cell. 2012;11(2):253-63.]. These cells were differentiated into NSCs as described [Ring KL, et al. Genomic Analysis Reveals Disruption of Striatal Neuronal Development and Therapeutic Targets in Human Huntington's Disease Neural Stem Cells. Stem Cell Reports. 2015;5(6):1023-38]. Briefly, iPSCs were passaged with ReLesR (STEMCELL technologies) and cell clumps were cultured in a low attachment petri-dish (coated with 0,1% agarose) in ES medium without bFGF (Embryonic Stem culture medium: KnockOut DMEM/F12 (Gibco) supplemented with 20% KnockOut Serum Replacement (Gibco), 2.48 mM L-glutamine, 1X NEAA, 15.4 mMHEPES, 50 µM β-mercaptoethanol, 100U/ml penicillin, 100 µg/ml streptomycin and 4ng/ml basic Fibroblast Growth Factor (bFGF) (PeproTech, 100-18B). Every 2 days, 25% of ES medium was replaced by EB differentiation medium (DMEM supplemented with 20% FBS, 1X Non Essential Amino-acids, 50 µM β-mercaptoethanol, 100U/ml penicillin and 100 µg/ml streptomycin). At day 8, 100% of the culture medium was embryoid body (EB) medium. At day 10, the EBs were attached to poly-L-Omithine/Laminin (pO/L, Sigma-Aldrich P4957 and L2020, respectively) coated dishes in Neural Induction medium (DMEM/F12 supplemented with 1X N2 (Gibco), 100 U/ml penicillin and 100 µg/ml streptomycin) and 25 ng/ml bFGF. The culture medium was replaced every 2 days. After 10-12 d, rosettes were picked using the STEMdiff^{™} Neural Rosette Selection Reagent (STEMCELL Technologies) and plated onto pO/L-coated plates in complete neural proliferation medium (NPM) (Neurobasal medium, 1X B27-supplement (Gibco), 2 mM L-glutamine, 25 ng/ml bFGF, 10 ng/ml leukemia inhibitory factor (LIF) (Peprotech, 300-05), 100U/ml penicillin, 100 µg/ml streptomycin). The level of differentiation into NSCs was tested by immunofluorescence using antibodies against the NSC markers Nestin (Sigma-Aldrich, 1:200) and SOX1 (Sigma-Aldrich, 1:50) and the iPSC marker OCT3/4 (Pierce antibodies, 1:500). The level of differentiation into NSCs across all experiments was at least 98%. The iPSC lines were verified for genome integrity prior to performing experiments using multi-color FISH analysis carried out by Applied Stemcell Inc. (Menlo Park, CA). To generate pre-patterned Activin A NSCs, the NSCs generated using above protocol were consistently maintained in 25 ng/ml Activin A (Peprotech) after EB stage starting at day 10.

To generate MSNs from the aforementioned NSCs (example 5, Figure 9), cocktails of small molecules were used across three weeks of cell culture as described before [Telezhkin V1, et al. Forced cell cycle exit and modulation of GABAA, CREB, and GSK3β signaling promote functional maturation of induced pluripotent stem cell-derived neurons. Am J Physiol Cell Physiol. 2016 Apr 1;310(7):C520-41.]. Differentiation of MSNs was assessed using immunofluorescence using antibodies against β3-Tubulin (Sigma-Aldrich, 1:500) and GABA (Sigma-Aldrich, 1:500). On average, >80% of cells in our differentiated population were MSNs (expressing β3-Tubulin and GABA).

Non-isogenic HD and control iPSC lines ND41656 (CAG 57), ND42222 (CAG 109), ND42241 were obtained from Coriell Repository, and MN08i-33114.B line from WiCell. NSC lines were generated using PSC neural induction medium (Life Technologies) as per instructions in the manual. Briefly, iPSCs cultured in mTeSR were harvested using 1 mg/ml collagenase. The colonies were transferred to a 60 mm dish coated with Matrigel (1:60 dilution, BD Biosciences) and cultured in PSC neural induction medium supplemented with 1 µM LDN-193189 and 10 µM SB431542 for 7 days to induce neuroepithelial fate. These cells were then harvested and expanded in neural expansion medium (PSC neural induction medium and DMEM/F12 medium (1:1), 100U/ml penicillin and 100 µg/ml streptomycin, and 2 mM L-Glutamine) supplemented with 25 ng/ml bFGF.

### Gene expression analysis

Total RNA was isolated from cells using the RNeasy kit (Qiagen), and DNase treated using the DNA-free DNA removal kit according to the manufacturer's instructions Kit (Ambion). Equal amounts of total RNA (1 µg) was reverse-transcribed using the RevertAID First Strand cDNA synthesis kit (Thermo Fisher scientific, K 1622), according to the manufacturer's instructions. The first strand cDNA was diluted and used as template in the real-time quantitative-PCR analysis. The LightCycler 480 Real-Time PCR System was used to perform the qRT-PCR using GoTaq qPCR Master Mix (Promega, A6002). qRT PCR experiments were performed in triplicate using the following primers: *FOXO3:* Forward: 5'-AGGGAGTTTGGTCAATCAGAA-3' (SEQ ID No. 1), Reverse: 5'- TGGAGATGAGGAATCAAAGTT-3' (SEQ ID No. 2); Ryk: Forward: 5'-CCACTTCTACGCGTGTGTTT-3' (SEQ ID No. 3), Reverse: 5'-GCCCTTGGGAACTACTGC-3' ((SEQ ID No. 36); *p16^{INK4}*: Forward: 5'-CCAACGCACCGAATAGTTACG-3'(SEQ ID No. 4), Reverse: 5'-GCGCTGCCCATCATCATG-3' (SEQ ID No. 5); *p14^{ARF}*: Forward: 5'-CCCTCGTGCTGATGCTACTG-3' (SEQ ID No. 6), Reverse: 5'-CATCATGACCTGGTCTTCTAGGAA-3' (SEQ ID No. 7); *CDKN2AIP:* Forward: 5'-GTGTATAGGGTCGGCCATCAA-3' (SEQ ID No. 8), Reverse: 5'-CCTGCCGTTGTTACCTGAGAG-3'(SEQ ID No. 9); *SERTAD1*: Forward: 5'-CTCAAGCTCCACCACAGCCT-3' (SEQ ID NO. 10), Reverse: 5'-AGTGTTCACGACCAGCACCA-3' (SEQ ID NO. 11); *ETS2:* Forward: 5'-CTGGGCATTCCAAAGAACCC-3' (SEQ ID NO. 12), Reverse: 5'-CCAGACTGAACTCATTGGTGG-3' (SEQ ID NO. 13); *ETS1* Forward: 5'-GGGAGGACCAGTCGTGGTAAA-3' (SEQ ID NO. 14), Reverse: 5'-CACGCTGCAGGCTGTTGAAAG-3' (SEQ ID NO. 15); *p21^{CIP1}*: Forward: 5'-CACCGAGGCACTCAGAGGAG-3'(SEQ ID NO. 16), Reverse 5'-CCGCCATTAGCGCATCACAG-3'(SEQ ID NO. 17); *p27^{KIP1}.* Forward: 5'-TAATTGGGGCTCCGGCTAACT-3'(SEQ ID NO. 18), Reverse: 5'-TGCAGGTCGCTTCCTTATTCC-3'(SEQ ID NO. 19); *HRPT.* Forward: 5'-ATGCTGAGGATTTGGAAAGG-3'(SEQ ID NO. 20) Reverse: 5'-CTCCCATCTCCTTCATCACA-3'(SEQ ID NO. 21); *ACTB:* Forward: 5'-CCAACCGCGAGAAGATGA -3'(SEQ ID NO. 22), Reverse: 5'-CCAGAGGCGTACAGGGATAG-3'(SEQ ID NO. 23); FOSB: Forward:5'-TGACAGTGTTATCCCAAGACCC-3' (SEQ ID NO. 34), Reverse: 5'-CCAGCAGGACGGCATCA-3' (SEQ ID NO. 35). QRT-PCR was performed at 95 °C for 10 min, followed by 40 cycles at 95 °C for 15 sec, 60 °C for 30 sec, and 72 °C for 30 sec. Data were analyzed using the LightCycler 480 software (Roche) and advanced relative quantification method. Gene expression was quantified by the mean cycle threshold (Ct) value for triplicate measurements. Target gene expression was normalized to two housekeeping genes (HPRT and ACTB) according to the 2-ΔΔCt formula. Statistical analyses (2-way ANOVA and t-tests) were performed using GraphPad Prism v6.

For biochemical analysis of Activin A and MSNs total RNA was isolated from NSCs and MSNs using ISOLATE II RNA Mini Kit (Bioline). cDNA was prepared from 1 µg of RNA in a total reaction volume of 20 µl using the SensiFAST cDNA synthesis kit (Bioline). RT-PCR reactions were setup in a 384-well format using 2X SensiFAST Probe No-ROX kit (Bioline) and 1 µl cDNA per reaction in a total volume of 10 µl. RT-PCR was performed on the Roche LightCycler 480 instrument. For quantification, the threshold cycle, Ct, of each amplification was determined by using the second derivative maximum method. The 2^{-ΔΔCt} method was used to determine the relative expression levels of each gene normalized against the housekeeping gene b-actin. The primers used were as follows: *p16^{INK4a}*: Forward: 5'-CAGCAGCATGGAGCCTTC-3'(SEQ ID NO. 24), Reverse: 5'-CGTAACTATTCGGTGCGTTG-3'(SEQ ID NO. 25), Probe 67 and Forward: 5'-CTGCCCAACGCACCGAATA-3'(SEQ ID NO. 26), Reverse: 5'-GCTGCCCATCATCATGACCT-3'(SEQ ID NO. 27), Probe FAM; FOXO3: Forward: 5'-CTTCAAGGATAAGGGCGACA-3' (SEQ ID NO. 28), Reverse: 5'-CGACTATGCAGTGACAGGTTG-3'(SEQ ID NO. 29), Probe 11; MMP3: Forward 5'-GCTGATATAATGATCTCTTTTGCAGT-3' (SEQ ID NO. 30), Reverse: 5'-CATAGGCATGGGCCAAAA-3' (SEQ ID NO. 31), Probe 85.

### Immunofluorescence analysis and quantification of p16^{INK4a}, γH2AX and HMGB1

NSCs plated (and differentiated into MSNs) in 8-well Nunc Lab-Tek II Chamber Slides (Thermo Fisher Scientific) were fixed with 4% paraformaldehyde for 15 min at room temperature (RT), and washed twice with PBS. Cells were permeabilized with 0.25% Triton X-100 (Sigma-Aldrich) in PBS for 15 min at RT, then washed twice with PBS. Blocking was performed using 5% donkey serum and 1% BSA in PBS for 30 min at RT. Cells were washed with PBS, and incubated overnight at 4 °C with primary antibody, washed with PBS three times, and incubated with fluorescent secondary antibody in the dark for 2 h at RT. Following three washes with PBS, coverslips were mounted using ProLong Gold antifade with DAPI (Thermo Fisher Scientific). Slides were cured for 24 h in the dark at RT, and imaging performed on Nikon Eclipse Ti-U microscope using the Plan Apo λ 20X/0.75 objective. Primary antibodies directed against p16^{INK4a} (Abcam, ab108349), HMGB1 (Abcam, ab18256), and Nestin (SCBT, sc-23927) were used at a dilution of 1:100. The primary antibody directed against γH2AX (Merck Millipore, 05-636) was used at a dilution of 1:1000. Secondary Alexa Fluor antibodies were purchased from Invitrogen. Image analysis was performed using the Gen5 software. TIFF images were converted to monochrome images and single cell analysis was performed using DAPI-stained nuclei to define the region of interest.

### Senescence-associated ß-galactosidase (SA-ß-gal) staining

NSCs were cultured as described above with the addition of 25 ng/ml Activin A (Peprotech, AF-120-14E). NSCs were stained using the Senescence staining kit (#9860, Cell Signaling Technology). Nuclei were stained with DAPI, and coverslips were mounted as described above. Images were captured using the Lionheart FX Automated Microscope and a 10X Plan Fluorite WD 10 NA 0.3 objective. Image analysis was performed using the Gen5 software. TIFF images were converted to monochrome images and single cell analysis was performed using DAPI-stained nuclei to define the region of interest and average SA-β-gal intensity/cell was quantified.

### Differentiation of human NSCs into MSNs

60 mm dishes or 6-well plates were coated with 100 µg/ml poly-D-lysine (Sigma-Aldrich, P6407) followed by Matrigel (1:60, Corning) coating. NSCs were plated and cultured in NPM. When confluent, NSCs were treated with Synaptojuice A medium for 1 week followed by Synaptojuice B medium for 10 d at 37° C [71]. 25 ng/ml Activin A was added to both Synaptojuice A and Synaptojuice B media. Half media change was performed every 2 days. The resulting MSNs were characterized by immunofluorescence using antibodies against the following: β-III-tubulin (SCBT, sc-80005), DARPP-32 (SCBT, sc-11365), Calbindin D-28K (Sigma-Aldrich, C9848), GABA (Sigma-Aldrich, A2052), MAP2 (EMD Millipore, AB5622). MSNs labeled positively for these markers. DARPP-32 expression was also determined by RT-PCR.

### Transfection of human neural stem cells

FOXO3 siRNAs (ON-TARGET plus SMART pool, L-003007-00-0020), ETS1 siRNAs (ON-TARGET plus SMART pool, L-003887-00-0005), ETS2 siRNAs (ON-TARGET plus SMART pool, L-003888-00-0005) and negative control siRNAs (ON-TARGET plus Non-targeting Control pool, D-001810-10-20) were obtained from Dharmacon (GE-Healthcare). Previously validated siRNAs targeting exon 1 of CDKN2A were p16^{INK4A} siRNA-1 (SEQ ID No. 32 :5'-AACGCACCGAATAGTTACGGT-3') [Kan CY et al. Endothelial cell dysfunction and cytoskeletal changes associated with repression of p16(INK4a) during immortalization. Oncogene. 2012;31(46):4815-27helial cell dysfunction and cytoskeletal changes associated with repression of p16(INK4a) during immortalization. Oncogene. 2012;31(46):4815-27] and p16^{INK4A} siRNA-2 (SEQ ID No. 33 : 5'-CUGCCCAACGCACCGAAUA-3') [Lejeune FX, et al. Large-scale functional RNAi screen in C. elegans identifies genes that regulate the dysfunction of mutant polyglutamine neurons. BMC Genomics. 2012;13:91. Epub 2012/03/15] and non-specific control 47% CG siRNA were obtained from Eurofins Genomics. Human NSCs were transfected using the Neon System 100 µl kit (Life Technologies MK10096) according to the manufacturer's guidelines. Briefly, cells were harvested with Stempro Accutase (Life Technologies, A1110501), washed with DPBS and resuspended in Buffer R at 2 × 10⁷ cells/ml. 2 × 10⁶ cells were mixed with 250 nM siRNA. Conditions used for the electroporation were pulse voltage 1400 V, pulse width 20 ms and 2 pulses. Cells were seeded in 6- well matrigel-coated plates with 2 ml pre-warmed growth medium without antibiotics, and incubated at 37 °C. 48 h after transfection, complete NPM was replaced with medium without bFGF and LIF for 6 h before total RNA extraction.

### Cell proliferation assays

Human NSCs were seeded on 24-well plates at 0.5-1 × 10⁵ cells per well, 6 wells for per genotype. After 1, 2, 3, 4 and 5 days at 37 °C and 5% CO₂, the medium was replaced with 500 µl fresh medium containing 10% v/v AlamarBlue^{®} reagent (ThermoFisher Scientific, DAL1025) according to the manufacturer's protocol. The plates were then incubated at 37° C for 3 hours. 100 µl from each well was transferred to a 96-well plate for reading. Fluorescence (excitation and emission wavelength of 550 and 595 nm) was measured using the Infinite^{®}F500 microplate reader (Tecan Genios). The 100% reduced form of AlamarBlue^{®}, (*i.e*., medium containing 10% v/v AlamarBlue^{®} autoclaved at 121° C for 15 min) were used as positive control. Wells without cells with culture medium containing 10% v/v AlamarBlue were used as negative controls. The relative fluorescence intensity for each genotype and each day was calculated as the AlamarBlue^{®} fluorescence signal of the sample at day X minus the signal of the negative control. Statistical analyses (2-way ANOVA) were performed using Prism v6.

### Cellular vulnerability assays

Human NSCs were subj ected to 24 h of growth factor deprivation as performed 48 h after cell transfection (by electroporation, as described above), after which cell viability and caspase-3/7 activity were detected using the ApoLive-Glo Multiplex Assay (Promega, G6410) according to the manufacturer's instructions. Briefly, 10 µl of reagent (GF-AFC substrate) were added to each well, and gently mixed with a plate shaker for 30 seconds. After incubation for 30 min at 37° C, fluorescence was measured using the plate-reader FLUOstar Optima (Ex at 360 nm, Em at 490 nm, BMG Labtech). Then, 50 µl of Caspase-Glo^{®} 3/7 reagent was added to each well, and gently mixed for 30 seconds. These plates were then incubated at room temperature for 30 min and luminescence of each sample measured using the plate-reader FLUOstar Optima (BMG Labtech). Caspase-3/7 assays were performed using five replicates per point and data was expressed as Caspase-3/7 activity (RLU) divided by cell viability (RFU). Statistical analyses (Student's t-test) were performed using GraphPad Prism v6.

### Statistics

Statistics were performed using Student's t-tests, one-way ANOVA with correction for multiple testing by Tukey's Multiple Comparison Test or two-way ANOVA. All experiments were repeated at least three times. P < 0.05 was considered significant.

### Example 2: Prepatterned HD NSCs show cellular senescence features in striatal neurons

Given that p16^{INK4a} is a key effector of cellular senescence in response to stress [Baker DJ, Childs BG, Durik M, Wijers ME, Sieben CJ, Zhong J] , our results (Fig 1 A to D) raise the possibility that one outcome of FOXO3 target reprogramming is to oppose cellular senescence features acquired by HD NSCs in the course of neuronal differentiation. We therefore assayed p16^{INK4a} expression in Activin A dorsoventral prepatterned C116 and HD cells (Fig 2). To this end, we used Activin A-induced dorsoventral prepatterning as Activin A is reported to efficiently direct striatal projection neuron differentiation of human iPSCs [Biswas SC, Zhang Y, Iyirhiaro G, Willett RT, Rodriguez Gonzalez Y, Cregan SP, et al. Sertad1 plays an essential role in developmental and pathological neuron death. J Neurosci. 2010;30(11):3973-82].*p16^{INK4a}* mRNA levels were increased in HD compared to C116 prepatterned NSCs (Fig 2A). Correspondingly, the levels of p16^{INK4a} positive cells were more prevalent in prepatterned HD compared to C116 NSCs, as measured by ICC (Fig 2B and 2C). In addition, senescence-associated β-galactosidase (SA-β-gal) activity, a widely-used putative senescence marker, was more abundant in HD compared to C116 NSCs (Fig 2D, 2E and 2F). Next, we tested for cellular senescence in NSCs derived from additional non-isogenic HD iPSC (namely, ND41656 and ND42222) and control iPSC (namely, MIN08i-33114.B and ND42241) lines. Consistent with our previous findings with the isogenic NSC model, we report robust increase in p16^{INK4a} expression (Fig 3A and 3B) and elevated SA-β-galactosidase activity (Fig 3C) in the non-isogenic HD NSC lines. These results corroborate that cellular senescence in HD NSCs can be directly attributed to the presence of the HD mutation and exclude the possibility that the senescence phenotype exhibited by isogenic HD NSCs is a result of clonal dependency.

We also tested if p16^{INK4a} levels were altered in HD MSNs derived from HD NSCs (Fig 4A). *p16^{INK4a}* mRNA levels were elevated in HD MSNs, which was also true for *FOXO3* and Ryk mRNA levels (Fig 4B). The increase in *p16^{INK4a}* mRNA levels (~5-fold; Fig 4A) was greater in magnitude than the increase in human HD NSCs (~1.7-fold; Fig 2A) and was accompanied by increased p16^{INK4a} immunostaining (Fig 4C-E). We also tested for other markers of cellular senescence, including elevated mRNA levels of *CDKN1A* encoding the p53 responsive CDK inhibitor p21^{CIP1}, *CDKN1B* encoding the CDK inhibitor p27^{KIP1}, a gene encoding a member of the AKT-FOXO pathway, and *MMP3* encoding a matrix metalloproteinase (Fig 7). Under basal conditions, *p21^{CIP1}* mRNA levels were decreased (Fig 7A), *p27^{KIP1}* mRNA levels were unchanged (Fig 7B) *andMMP-3* mRNA levels were increased (Fig 7C) in HD NSCs compared to C116 NSCs. Thus, HD NSCs show increased levels of several markers of cellular senescence (p16^{INK4a}, MMP-3, SA-β-gal). Additionally, human HD MSNs showed decreased levels of nuclear HMGB1, which relocalizes to the extracellular space in senescent cells [Li J, Dissection of CDK4-binding and transactivation activities of p34(SEI-1) and comparison between functions of p34(SEI-1) and p16(INK4A). Biochemistry. 2005;44(40):13246-56] ; this decrease in nuclear HMGB1 was not observed in human HD NSCs (Fig 7D and 7E). Collectively, these results suggest that the differentiation of NSCs into striatal neurons is accompanied by increasingly pronounced features of cellular senescence in HD.

### Example 3: p16^{INK4a} affect stress vulnerability in human HD NSCs

FOXO factors are important regulators of stem cell homeostasis in several tissues [Ohtani N, et al. Opposing effects of Ets and Id proteins on p16INK4a expression during cellular senescence. Nature. 2001;409(6823):1067-70 , Arber C, , et al. Activin A directs striatal projection neuron differentiation of human pluripotent stem cells. Development (Cambridge, England). 2015;142(7):1375-86 aniczek JR, Kelly C, Noakes Z, et al. Activin A directs striatal projection neuron differentiation of human pluripotent stem cells. Development (Cambridge, England). 2015;142(7):1375-86] and previous studies suggested that p16^{INK4a} may act downstream to FOXO factors for regulating the maintenance of the hematopoietic stem cell pool [Ohtani N, et al. Opposing effects of Ets and Id proteins on p16INK4a expression during cellular senescence. Nature. 2001;409(6823): 1067-70]. Our results (Fig 1) suggest FOXO3 activity could oppose the p16^{INK4a} increase by repressing ETS2 in stressed human HD NSCs. Additionally, human HD NSCs show increased levels of cellular senescence markers, which increases further as they differentiate into neurons (Fig 2-4), and p16^{INK4a} is thought to promote aging phenotypes in stem cells [Davalos AR, et al. p53-dependent release of Alarmin HMGB1 is a central mediator of senescent phenotypes. J Cell Biol. 2013;201(4):613-29]. Thus, FOXO3 activity in HD NSCs might oppose the effects of p16^{INK4a} on the homeostasis of the neural stem cell pool in HD. Therefore, we tested the effects of silencing *FOXO3* or *p16^{INK4a}* on cell doubling times in culture and cell vulnerability as measured by levels of caspase-3/7 activation after serum deprivation.

Human HD NSCs divided more slowly compared to C116 NSCs (Fig 5A). Reducing *FOXO3* (Fig 6A) retarded the growth of human HD NSCs (Fig 5B, left panel) with no change detected in *HTT* expression (Fig 6C, left panel) and a trend (not significant) toward reduced growth of C116 NSCs (Fig 5B, right panel). These findings suggest FOXO3 promotes the growth of human HD NSCs. Reducing *p16^{INK4a}* (Fig 6B) slightly increased the growth of both HD (Fig 5C, left panel) and C116 (Fig 5C, right panel) NSCs, without changing *HTT* expression (Fig 6C, right panel), suggesting that *p16^{INK4a}* normally restrains the growth of human NSCs, regardless of the *HTT* genotype. Considering our data on the FOXO3-ETS2-p16^{INK4A} pathway (Fig 1D), these results suggest that p16^{INK4A} does not significantly impact the dynamics of the NSC pool in HD.

In cellular vulnerability assays, reducing *FOXO3* expression (Fig 6A) strongly potentiates the mortality of HD NSCs with no effect in C116 cells (Fig 5D, left panel), suggesting that FOXO3 promotes the survival of human HD NSCs. In contrast, reducing *p16^{INK4a}* expression (Fig 6B) decreased the mortality of HD NSCs, with no effect detected in C116 cells (Fig 5D, right panel), suggesting that increased *p16^{INK4a}* in human HD NSCs is deleterious. Considering our data on the FOXO3-ETS2-p16^{INK4a} pathway, these results suggest that FOXO3 activity opposes the detrimental effects of p16^{INK4a} on the vulnerability and survival of the neural stem cell pool in HD.

### Example 4: Chronic genetic inhibition of p16^{INK4a} decreases the persistence of DNA Damage Repair (DDR) machinery in HD72Q NSC.

After acute oxidative stress (400 mM H2O2 for 1 hour), the levels of yH2AX, a marker of the DDR machinery that recognizes DNA Double Strand Breaks (DSB), are persistently higher in HD72Q/19Q NSCs compared to C116 cells (Figure 8).

Additionally, chronic genetic inhibition of p16^{INK4a} decreases the persistence of γH2AX nuclear puncta in HD cells recovering from acute oxidative stress (Figure 8). It has been described that the persistence of DDR might be a pathogenic phenomenon (could mark DNA scars) (DNA-SCARS: distinct nuclear structures that sustain damage-induced senescence growth arrest and inflammatory cytokine secretion.), suggesting that p16^{INK4a} inhibition alleviate pathogenicity in this human NSC context (Rodier F, Muñoz DP, Teachenor R, Chu V, Le O, Bhaumik D, Coppé JP, Campeau E, Beaus6jour CM, Kim SH, Davalos AR, Campisi J. J Cell Sci. 2011 Jan 1;124(Pt 1):68-81).

### Example 5. Chronic genetic inhibition of p16^{INK4a} decreases the hyperactivity of HD MSNs in vitro.

HD neurons hyperactivity (leading to excito-toxicity) has been described in different models (Excitotoxic neuronal death and the pathogenesis of Huntington's disease. Estrada Sánchez AM1, Mejía- Toiber J, Massieu L. Arch Med Res. 2008 Apr;39(3):265-76; Amoux I, Willam M, Griesche N, et al. Metformin reverses early cortical network dysfunction and behavior changes in Huntington's disease. Elife. 2018;7:e38744. Published 2018 Sep 4. doi: 10.7554/eLife.38744). Here we used iPSC-derived MSNs to study this phenomenon. Moreover, we linked neuronal activity with DNA Damage Repair capacities using Etoposide, a topoisomerase II inhibitor that generates DSBs.. DSBs are thought to open chromatin and make it accessible to the transcriptional machinery, thus activating transcription at specific genomic sites. The ability to repair DSBs is linked to the normal decrease of transcription at these genomic sites.

Etoposide has been shown to activate neurons *in vitro* as some genes (such as early response genes) are primed for transcription activation by the presence of topoisomerase II at their promoter (Activity-Induced DNA Breaks Govern the Expression of Neuronal Early-Response Genes. Madabhushi R, Gao F, Pfenning AR, Pan L, Yamakawa S, Seo J, Rueda R, Phan TX, Yamakawa H, Pao PC, Stott RT, Gjoneska E, Nott A, Cho S, Kellis M, Tsai LH. Cell. 2015 Jun 18;161(7):1592-605). As a reporter of neuronal activity, we monitored the expression of FOSB, an early response gene.

p16^{INK4a} inhibition using shRNAs attenuates the differences between HD and control (C116) MSNs, indicating that HD cells treated with shp16 (and shCDKN2A) may normalize their Etoposide response and also their FOSB levels in basal (untreated) conditions (Figure 9).

Collectively, these data corroborate a model in which the inhibition of p16^{INK4a} not only protects human HD NSCs from chronic and maladaptive senescence responses but is also able to restore a normal activity in human HD medium spiny neurons.

## Claims

1. A p 16^{INK4a} inhibitor for use in preventing and/or treating Huntington's disease (HD) in a subject in need thereof.

2. The p16^{INK4a} inhibitor for use according to claim **1**, wherein said inhibitor is a nucleic acid or a peptide, a small compound molecule.

3. The p 16^{INK4a} inhibitor for use according to claim **2**, wherein the nucleic acid encodes an RNA interfering with p 16^{INK4a} such as a small interfering RNA (siRNA), a small hairpin RNA (shRNA), a micro RNA (miRNA), a non-coding RNA, a deoxyribosyme, an antisense oligonucleotide, ribozymes DNAzymes, modified or synthetic DNA or RNA degradation-resistant polynucleosides amides, peptide nucleic acids (PNAs), locked nucleic acids (LNAs), other nucleobase-containing polymers, aptamers or a polynucleotide for targeted gene editing or any combination thereof.

4. The p16^{INK4a} inhibitor for use according to claim **2**, wherein the peptide is chosen among the group comprising a ligand, an inhibitor of kinase, a small compound molecule such as PPARγ antagonist or such as a retinoid X receptor (RXR) antagonist, small molecule SIRT1 activators, a compound able to stimulate the activity of FOXO factors and AMPK activators.

5. The p16^{INK4a} inhibitor for use according to claim **4**, wherein the ligand is an antibody, a Fab, a Fab', a F(ab')2, a Fv, a dsFv, a scFv, a diabody, a triabody, a tetrabody, an aptamer or a VHH domain.

6. A composition for use in treating and/or preventing HD in a subject in need thereof, wherein said composition comprises at least one p 16^{INK4a} inhibitor according to any one of claims **1** to **5.**

7. The composition for use according to claim **6**, wherein the composition is a pharmaceutical composition and further comprises at least one pharmaceutically acceptable excipient.

8. The composition for use according to any one of claims **6** or **7**, further containing a nucleic acid sequence encoding a peptide for cell-specific targeting and/or a nucleic acid enabling a cell-specific expression.

9. The composition for use according to any one of claims **6** to **8**, further comprising one or more active agent(s) for treating HD and/or side effects of said active agent(s).

10. A medicament for use in treating and/or preventing HD in a subject in need thereof, wherein said medicament comprises at least one p16^{INK4a} inhibitor according to any one of claims **1** to **5** or the composition according to any one of claims **6** to **9**, wherein said medicament is to be administered to the subject in a therapeutically effective amount.

11. The p16^{INK4a} inhibitor, the composition or the medicament for use according to any one of the preceding claims, wherein the subject is diagnosed with HD, presents a genetic predisposition to HD or is affected with HD.

12. The p16^{INK4a} inhibitor, the composition or the medicament for use according to claim **11,** wherein the subject is diagnosed with HD.

## Patentansprüche

1. p16^{INK4a}-Inhibitor zur Verwendung bei der Vorbeugung und/oder Behandlung der Huntington-Krankheit (HD) bei einem Patienten, der dessen bedarf.

2. p16^{INK4a}-Inhibitor zur Verwendung nach Anspruch **1**, wobei der Inhibitor eine Nukleinsäure oder ein Peptid, ein kleines Verbindungsmolekül ist.

3. p16^{INK4a}-Inhibitor zur Verwendung nach Anspruch **2**, wobei die Nukleinsäure eine mit p16^{INK4a} interferierende RNA kodiert wie etwa eine kleine interferierende RNA (siRNA), eine Small hairpin RNA (shRNA), eine Mikro-RNA (miRNA), eine nicht kodierende RNA, ein Desoxyribosym, ein Antisense-Oligonukleotid, Ribozyme, DNAzyme, modifizierte oder synthetische gegen DNA- oder RNA-Abbau resistente Polynukleotide, Amide, Peptidnukleinsäuren (PNAs), gesperrte Nukleinsäuren (LNAs), andere Nukleobasen enthaltende Polymere, Aptamere oder ein Polynukleotid für die gezielte Genbearbeitung oder eine beliebige Kombination davon.

4. p16^{INK4a}-Inhibitor zur Verwendung nach Anspruch **2**, wobei das Peptid ausgewählt ist aus der Gruppe, die einen Liganden, einen Inhibitor der Kinase, ein kleines Verbindungsmolekül wie einen PPARy-Antagonisten oder wie einen Retinoid-X-Rezeptor (RXR)-Antagonisten, niedermolekulare SIRT1-Aktivatoren, eine Verbindung, die in der Lage ist, die Aktivität von FOXO-Faktoren zu stimulieren, und AMPK-Aktivatoren umfasst.

5. p16^{INK4a}-Inhibitor zur Verwendung nach Anspruch **4**, wobei der Ligand ein Antikörper, ein Fab, ein Fab`, ein F(ab')2, ein Fv, ein dsFv, ein scFv, ein Diabody, ein Triabody, ein Tetrabody, ein Aptamer oder eine VHH-Domäne ist.

6. Zusammensetzung zur Verwendung bei der Behandlung und/oder Vorbeugung der HD bei einem Patienten, der dessen bedarf, wobei die Zusammensetzung mindestens einen p16^{INK4a}-Inhibitor nach einem der Ansprüche 1 bis 5 umfasst.

7. Zusammensetzung zur Verwendung nach Anspruch 6, wobei die Zusammensetzung eine pharmazeutische Zusammensetzung ist und ferner mindestens einen pharmazeutisch verträglichen Exzipienten umfasst.

8. Zusammensetzung zur Verwendung nach einem der Ansprüche 6 oder 7, ferner enthaltend eine Nukleinsäuresequenz, die ein Peptid für zellspezifisches Targeting kodiert, und/oder eine Nukleinsäure, die eine zellspezifische Expression ermöglicht.

9. Zusammensetzung zur Verwendung nach einem der Ansprüche 6 bis 8, ferner umfassend einen oder mehrere Wirkstoffe zur Behandlung von HD und/oder Nebenwirkungen des Wirkstoffs bzw. der Wirkstoffe.

10. Medikament zur Verwendung bei der Behandlung und/oder Vorbeugung der HD bei einem Patienten, der dessen bedarf, wobei das Medikament mindestens einen p16^{INK4a}-Inhibitor nach einem der Ansprüche 1 bis 5 oder die Zusammensetzung nach einem der Ansprüche 6 bis 9 umfasst, wobei das Medikament dem Patienten in einer therapeutisch wirksamen Menge zu verabreichen ist.

11. p16^{INK4a}-Inhibitor, Zusammensetzung oder Medikament zur Verwendung nach einem der vorhergehenden Ansprüche, wobei bei dem Patienten HD diagnostiziert wird, eine genetische Prädisposition für HD aufweist oder von HD betroffen ist.

12. p16^{INK4a}-Inhibitor, Zusammensetzung oder Medikament zur Verwendung nach Anspruch 11, wobei bei dem Patienten HD diagnostiziert wird.

## Revendications

1. Inhibiteur de p16^{INK4a} pour son utilisation dans la prévention et/ou le traitement de la maladie de Huntington (MH) chez un sujet en ayant besoin.

2. Inhibiteur de p16^{INK4a} pour son utilisation selon la revendication 1, dans lequel ledit inhibiteur est un acide nucléique ou un peptide, une petite molécule.

3. Inhibiteur de p16^{INK4a} pour son utilisation selon la revendication 2, dans lequel l'acide nucléique code pour un ARN interférant avec p16^{INK4a} tel qu'un petit ARN interférent (siRNA), un petit ARN en épingle à cheveux (shRNA), un micro ARN (miRNA), un ARN non codant, un désoxyribosyme, un oligonucléotide antisens, des ribozymes DNAzymes, des polynucléosides amides modifiés ou synthétiques résistants à la dégradation de l'ADN ou de l'ARN, des acides nucléiques peptidiques (PNA), des acides nucléiques verrouillés (LNAs), d'autres polymères contenant une nucléobase, des aptamères ou un polynucléotide pour l'édition ciblée de gènes ou toute combinaison de ceux-ci.

4. Inhibiteur de p16^{INK4a} pour son utilisation selon la revendication 2, dans lequel le peptide est choisi dans le groupe comprenant un ligand, un inhibiteur de kinase, une petite molécule composée telle qu'un antagoniste de PPAPγ ou telle qu'un antagoniste du récepteur X des rétinoïdes (RXR), des petites molécules activatrices de SIRT1, un composé capable de stimuler l'activité des facteurs FOXO et, des activateurs d'AMPK.

5. Inhibiteur de p16^{INK4a} pour son utilisation selon la revendication 4, dans lequel le ligand est un anticorps, un Fab, un Fab', un F(ab')2, un Fv, un dsFv, un scFv, un diabody, un triabody, un tétrabody, un aptamère ou un domaine VHH.

6. Composition pour son utilisation dans le traitement et/ou la prévention de la MH chez un sujet en ayant besoin, dans laquelle ladite composition comprend au moins un inhibiteur de p16^{INK4a} selon l'une quelconque des revendications 1 à 5.

7. Composition pour son utilisation selon la revendication 6, dans laquelle la composition est une composition pharmaceutique et comprend en outre au moins un excipient pharmaceutiquement acceptable.

8. Composition pour son utilisation selon l'une quelconque des revendications 6 ou 7, contenant en outre une séquence d'acide nucléique codant pour un peptide pour un ciblage cellulaire spécifique et/ou contenant un acide nucléique permettant une expression cellulaire spécifique.

9. Composition pour son utilisation selon l'une quelconque des revendications 6 à 8, comprenant en outre un ou plusieurs agent(s) actif(s) pour traiter la MH et/ou des effets secondaires dudit (desdits) agent(s) actif(s).

10. Médicament pour son utilisation dans le traitement et/ou la prévention de la MH chez un sujet en ayant besoin, dans lequel ledit médicament comprend au moins un inhibiteur de p16^{INK4a} selon l'une quelconque des revendications 1 à 5 ou la composition selon l'une quelconque des revendications 6 à 9, dans lequel ledit médicament doit être administré au sujet en une quantité thérapeutiquement efficace.

11. L'inhibiteur de p16^{INK4a}, la composition ou le médicament pour son utilisation selon l'une quelconque des revendications précédentes, dans lequel le sujet est diagnostiqué avec la MH, présente une prédisposition génétique à la MH ou est affecté par la MH.

12. L'inhibiteur de p16^{INK4a}, la composition ou le médicament pour son utilisation selon la revendication 11, dans lequel le sujet est diagnostiqué avec la MH.
